(19) Europäisches Patentamt
European Patent Office
Office européen des brevets

(11) **EP 4 574 088 A1**

(12) **EUROPEAN PATENT APPLICATION**

(43) Date of publication:
**25.06.2025 Bulletin 2025/26**

(21) Application number: **23218110.7**

(22) Date of filing: **19.12.2023**

(51) International Patent Classification (IPC):
**A61C 13/083** (2006.01)    **C04B 35/488** (2006.01)
**C04B 35/626** (2006.01)    **C04B 38/00** (2006.01)
**C04B 35/486** (2006.01)

(52) Cooperative Patent Classification (CPC):
(C-Sets available)
**C04B 35/488; A61C 13/0022; A61C 13/083;**
**C04B 35/486; C04B 35/4885; C04B 35/62645;**
**C04B 38/0067;** C04B 2111/00836; C04B 2201/20;
C04B 2201/52; C04B 2235/3225; C04B 2235/3246;
C04B 2235/6562; C04B 2235/6567; C04B 2235/72;
(Cont.)

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **Ivoclar Vivadent AG**
**9494 Schaan (LI)**

(72) Inventor: **REINSHAGEN, Jörg**
**75177 Pforzheim (DE)**

(74) Representative: **Maiwald GmbH**
**Elisenhof**
**Elisenstraße 3**
**80335 München (DE)**

(54) **DENTAL MILL BLANK, PROCESS FOR PREPARING THE SAME, AND USES THEREOF**

(57)    A dental mill blank is provided comprising an yttria-containing zirconia material. One embodiment provides a dental mill blank comprising an yttria-containing zirconia material, wherein the yttria-containing zirconia material has a fracture resistance of at least 6.0 MPa*m$^{-1/2}$, determined on a densely sintered test body of the yttria-containing zirconia material, and a biaxial flexural strength of at least 1200 MPa, determined on a densely sintered test body of the yttria-containing zirconia material. Furthermore, a process for preparing the dental mill blank, a process for preparing a dental restoration using the dental mill blank, and a dental restoration as such are provided.

**FIG. 3A**

**(Cont. next page)**

EP 4 574 088 A1

(52) Cooperative Patent Classification (CPC): (Cont.)
C04B 2235/77; C04B 2235/785; C04B 2235/95;
C04B 2235/96; C04B 2235/963; C04B 2235/9653;
C04B 2235/9661

C-Sets
**C04B 38/0067, C04B 35/488, C04B 38/0058**

**Description**

**TECHNICAL FIELD**

The present invention relates to a dental mill blank and to a process for preparing the dental mill blank. The present invention further relates to a dental restoration which is obtainable using the dental mill blank and to a dental restoration as such. The present invention further relates to a use of a zirconia powder for preparing a dental mill blank.

**BACKGROUND OF INVENTION**

Ceramic dental restorations are typically prepared from dental mill blanks. A dental mill blank is usually a porous and not perfectly sintered block or disc that is based on a ceramic material, like zirconia. The dental mill blank can be machined in a CAD/CAM process to prepare a ceramic dental restoration precursor. This dental restoration precursor has a shape of the final ceramic dental restoration but not yet its final density, and therefore not yet its final dimensions. The dental restoration precursor is then subjected to a sintering process to provide the ceramic dental restoration in its final density and dimensions.

Many commercially available dental mill blanks are based on yttria-stabilized zirconia materials. For example, dental mill blanks are available on the market that are based on 3 mol%-, 4 mol%-, or 5 mol%-yttria-stabilized zirconia materials (also referred to as 3Y-, 4Y-, and 5Y-TZP). These materials, and especially 3Y-TZP, can be used to provide a dental restoration with a comparatively high flexural strength. However, the fracture toughness or fracture resistance of said materials is not always satisfactory, especially for more challenging application of dental restorations such as multi-unit bridges or minimal-invasive restorations. Attempts were made in the art to increase fracture toughness/resistance of ceramic dental restorations by using cerium oxide-stabilized zirconia materials containing high amounts of aluminum oxides. However, said materials typically provide dental restorations which are fully opaque and/or yellowish, and which therefore do not fulfil the high esthetic requirements in dentistry with regard to color and translucency.

There is a continuing need in the art for a dental mill blank which is suitable for preparing a dental restoration which has improved mechanical properties, and especially which has an increased fracture toughness or fracture resistance. It is further desirable that the dental mill blank is suitable for preparing a dental restoration which provides appreciable color and translucency characteristics.

**OBJECTS AND SUMMARY OF THE INVENTION**

One object of the present invention is to provide a dental mill blank which is suitable for preparing a dental restoration having improved mechanical properties, compared to a prior art dental mill blank. One object of the present invention is to provide a dental mill blank which is suitable for preparing a dental restoration having a high flexural strength and a high fracture toughness or fracture resistance. One object of the present invention is to provide a dental mill blank which is suitable for preparing a dental restoration having a good balance of mechanical properties, like flexural strength and fracture toughness or fracture resistance, and optical properties, like color or translucency.

One or more of the above objects is solved by the dental mill blank, the process for preparing the dental restoration, the dental restoration according to the present invention, and/or the use according to the present invention.

One aspect of the present invention provides a dental mill blank comprising an yttria-containing zirconia material. One embodiment of the present invention provides a dental mill blank comprising an yttria-containing zirconia material, wherein the yttria-containing zirconia material has:

- a fracture resistance of at least 6.0 MPa*m$^{-1/2}$, determined on a densely sintered test body of the yttria-containing zirconia material, and
- a biaxial flexural strength of at least 1200 MPa, determined on a densely sintered test body of the yttria-containing zirconia material.

One embodiment of the present invention provides a dental mill blank comprising an yttria-containing zirconia material, wherein the yttria-containing zirconia material comprises yttria in an amount of at most 4.0 wt.%, based on the total weight of the yttria-containing zirconia material.

One finding of the present invention is that the dental mill blank according to the present invention is useful for preparing a zirconia ceramic, like a zirconia ceramic dental restoration, having good mechanical properties. In particular, a dental restoration having a high biaxial flexural strength and high fracture resistance can be prepared. It has also been found by the inventor that a dental mill blank comprising a zirconia material comprising at most 4.0 wt.% of yttria is particularly suitable for preparing a zirconia ceramic, like a zirconia ceramic dental restoration, with said mechanical properties. The dental mill blank is further suitable for preparing a zirconia ceramic, like a zirconia ceramic dental restoration, with a good balance of mechanical and optical properties. The dental mill blank may thus be suitable for preparing an esthetic dental restoration having a thin wall thickness which, for example, allows minimal-invasive treatments.

In another aspect of the present invention, a process is provided for preparing a dental mill blank according to an embodiment of the present invention. The process comprises the steps of:

- providing a zirconia powder,
- optionally treating the zirconia powder with one or more colorants,
- compacting the zirconia powder to provide a green body,
- pre-sintering the green body to provide a dental mill blank, and
- optionally pre-coloring the dental mill blank.

Another aspect of the present invention provides a use of a zirconia powder for preparing a dental mill blank, wherein the zirconia powder comprises

from 95.0 to 98.5 wt.%, preferably from 95.5 to 98.0 wt.%, like from 96.0 to 97.7 wt.%, of a combined amount of zirconia and hafnia,
from 1.5 to 4.0 wt.%, preferably from 2.0 to 3.8 wt.%, like from 2.2 to 3.4 wt.%, of yttria,
from 0.0 to 1.0 wt.%, preferably from 0.0 to 0.7 wt.%, like from 0.1 to 0.7 wt.% of aluminum oxide (e.g., from 0.1 to 0.4 wt.% or from 0.4 to 0.7 wt.%), and
from 0.0 to 0.3 wt.%, preferably from 0.0 to 0.1 wt.%, like from 0.0 to 0.05 wt.%, of other oxides, based on the total weight of the zirconia powder.

Another aspect of the present invention provides a process for preparing a dental restoration. The process comprises the steps of:

- machining a dental mill blank according to an embodiment of the present invention to provide a dental restoration precursor,
- optionally surface-treating the dental restoration precursor, and
- sintering the dental restoration precursor to provide a dental restoration.

Another aspect of the present invention provides a dental restoration. According to one embodiment, a dental restoration is provided which is obtainable by the process according to an embodiment of the present invention.

## DEFINITIONS

In the context of the present invention, the following terms have the following meaning:

"Dental mill blank" means a porous, solid, geometrically defined, three dimensional object of material, like a block or a disc, from which a dental restoration precursor can be machined by, e.g., cutting, milling, grinding, drilling, and the like. The machining is typically carried out using a computer-aided design/computer-aided manufacturing (CAD/CAM) process.

A "dental restoration precursor" as used herein refers to a work piece machined from a dental mill blank that already has the shape of a dental restoration but that has not yet been densely sintered, and therefore not yet its final dimensions.

"Dental restoration" as used herein refers to an article that is useful in the dental or orthodontic field for restoring, remodeling, supporting and/or restructuring a tooth or parts thereof or a group of teeth or parts thereof. A dental restoration may be, but is not limited to, a crown, a partial crown, an abutment, an abutment crown, an inlay, an onlay, a veneer, a shell or a bridge.

A "green body" is a molded body of ceramic powder that has not been subjected to a sintering or pre-sintering step, and that may contain an organic binder, an inorganic binder, or other additives. The molded body is usually prepared by compacting (e.g., pressing) the ceramic powder.

"Porous" as used herein means that a material has pores and is meant to comprise open-porous materials and closed-porous materials. An "open-porous" material is a material that has pores that are at least partially interconnected and that are at least partially accessible from the outside by, e.g., a flow of gas or liquid. An open-porous material as defined herein may also have pores that are not accessible from the outside by, e.g., a flow of gas or liquid. A "closed-porous" material is a material that is not open-porous and that has pores that are closed, i.e., pores that are not accessible from the outside by, e.g., by a flow of gas or liquid. "Porosity" is a measure of the void spaces, like pores, in a solid material, and is a fraction of the volume of the void spaces over the total volume of the solid material. It may be expressed as a percentage from 0 to 100%.

"Sintering" as used herein means the densification of a porous inorganic material to a less porous inorganic material having a higher density by subjecting the material to heat at an appropriate temperature for densification. The temperature is below the melting point of the main components of the inorganic material.

"Debinding" as used herein means subjecting a green body to heat in order to partially or fully remove or decompose organic binders, inorganic binders or thermally unstable components. Debinding can be carried out before or as part of a pre-sintering step.

"Pre-sintered" or "pre-sintering" as used herein means subjecting a green body to heat in order to promote an at least partial formation of sintering necks at connected particle boundaries in the material of the body and a thermal hardening of the material which can facilitate workability or machinability of the material. Pre-sintering may comprise a debinding of the green body. The relative densification from a green body to a pre-sintered material is typically 5% or less, relative to a dimension of the green body. A pre-sintered material typically has an open-porous structure that can be further densified when densely sintering the material in a subsequent sintering step. The density of pre-sintered materials, like pre-sintered zirconia materials, may be in the range of 45 to 70% relative to the theoretical density of the ceramic material. The temperature with which a material has been pre-sintered can be determined by the skilled person, e.g., by measuring the thermal expansion using a dilatometer.

"Densely sintered" or "densely sintering" as used herein means that a ceramic material has been sintered to a density of at least 98.5%, like at least 99.5% or at least 99.8%, of the theoretical density of the ceramic material. The density of the material can be determined by the Archimedes' method according to DIN EN 623-2, or by weighing the material and determining its volume geometrically. The theoretical density of a densely sintered material can be determined by the skilled person, e.g., based on the chemical composition of the material. Additionally or alternatively, the theoretical density of the ceramic material may be determined by grinding the ceramic material to a powder, e.g., having a volume-based median particle size in the range of 10 to 30 $\mu$m (e.g. 20 $\mu$m), and determining the density of the powder by pycnometry. The volume-based median particle size may be determined by laser diffraction, e.g., according to ISO 13320 (2009).

"Yttria-stabilized zirconia" as used herein means zirconia ($ZrO_2$) that is at least partially present in a tetragonal crystal phase or a cubic crystal phase and which has an amount of yttria ($Y_2O_3$) incorporated into its crystal lattice that is sufficient to at least partially prevent a transition of the tetragonal crystal phase and cubic crystal phase, respectively, into the monoclinic crystal phase during cooling down to room temperature. At room temperature, pure zirconia is present in its most stable crystal phase, the monoclinic crystal phase. When the temperature of zirconia is increased to approx. 1170°C, the monoclinic crystal phase transforms into the tetragonal crystal phase, and subsequently the tetragonal phase transform into the cubic crystal phase at approx. 2370°C. The incorporation of an appropriate amount of yttria into the crystal lattice of the zirconia at least partially stabilizes the tetragonal or cubic crystal phase of the zirconia, i.e., at least partially prevents a transition of the tetragonal crystal phase and cubic crystal phase, respectively, into the (at room temperature) more stable monoclinic crystal phase of zirconia.

A "pre-colored" dental mill blank means a dental mill blank that comprises coloring oxides in an amount and/or combination that is effective to impart a color (e.g., a color matching a natural color of tooth and/or matching a tooth color by VITA classical A1-D4® shade guide with VITA Bleached Shades manufactured by Vita Zahnfabrik or a similar dental shade guide system) to a dental restoration that is machined from the dental mill blank and then densely sintered.

Where the term "comprising" is used herein, it does not exclude that further non-specified elements are present. Where the term "essentially consisting of" is used herein, it is does not exclude that further non-specified elements are present that are not materially affecting the essential characteristics of the defined subject-matter. When e.g. a material is defined by its chemical composition, the material may contain unavoidable trace impurities (e.g., $SiO_2$, $CaO$, and/or $Na_2O$) in a sum of < 0.1 wt.%, even if this is not explicitly defined. For the purposes of the present invention, the terms "essentially consisting of" and "consisting of" are considered to be specific embodiments of the term "comprising of". Whenever the terms "including" or "having" are used, these terms are meant to be equivalent to "comprising" as defined above.

The term "obtained" does not necessarily mean to indicate that, e.g., an embodiment must be obtained by, e.g., a sequence of steps following the term "obtained" even though such a limited understanding is always included by the term "obtained" as a preferred embodiment.

Numbers defined herein are rounded to their last digit and are meant to encompass the range of rounding values according to established rounding rules. For example, the value 3 is meant to encompass the values in the range of 2.5 to 3.4, the value 1.5 is meant to encompass the values in the range of 1.46 to 1.54, and so on.

## BRIEF DESCRIPTION OF FIGURES

**FIG. 1A** and **Fig. 1B:** Light microscopic images (black/white) of a Vickers indentation (test load: 196.1 N) of a test body (densely sintered at 1250 °C) prepared from a dental mill blank obtained using powder P1.

**FIG. 1C** and **Fig. 1D:** Light microscopic images (black/white) of a Vickers indentation (test load: 196.1 N) of a test body (densely sintered at 1250 °C) prepared from a dental mill blank obtained using powder P2.

**FIG. 2A** and **Fig. 2B:** Light microscopic images (black/white) of a Vickers indentation (test load: 196.1 N) of a test body (densely sintered at 1450 °C) prepared from a prior art dental mill blank based on 3 mol%-$Y_2O_3$ stabilized zirconia (3Y-TZP).

**FIG. 2C** and **Fig. 2D:** Light microscopic images (black/white) of a Vickers indentation (test load: 196.1 N) of a test body (densely sintered at 1450 °C) prepared from a prior art dental mill blank based on 5 mol%-$Y_2O_3$ stabilized zirconia (5Y-TZP).

**FIG. 3A:** Image (black/white) of a dental mill blank obtained using binder-free powder P1 after milling of dental restoration precursors.

**FIG. 3B:** Image (black/white) of a dental mill blank obtained using binder-containing powder P1 after milling of dental restoration precursors.

**FIG. 4A:** Images (black/white) of dental restoration precursors before a final sintering step. The dental restoration precursor was obtained from a dental mill blank prepared using powder P1.

**FIG. 4B:** Images (black/white) of dental restoration after sintering at 1250°C. The dental restoration was obtained from a dental mill blank prepared using powder P1.

**FIG. 4C:** Images (black/white) of dental restoration after sintering at 1300°C. The dental restoration was obtained from a dental mill blank prepared using powder P1.

**FIG. 5:** Image (color) of a colored dental restoration with A3 dental shade after sintering at 1300°C. The dental restoration was obtained from a dental mill blank prepared using powder P1. Before final sintering, the milled dental restoration precursor was colored using brush infiltration technique and a commercial coloring liquid (IPS e.max ZirCAD LT Colouring Liquid A3 - available from Ivoclar Vivadent AG).

## DETAILED DESCRIPTION OF THE INVENTION

### I. Dental mill blank

### 1. The yttria-containing zirconia material

The dental mill blank according to the present invention comprises an yttria-containing zirconia material. The yttria-containing zirconia material is further characterized by its mechanical properties and/or its chemical properties.

1.1 Mechanical and optical properties

One embodiment of the present invention provides a dental mill blank comprising an yttria-containing zirconia material, wherein the yttria-containing zirconia material has:

-    a fracture resistance of at least 6.0 MPa*m$^{-1/2}$, determined on a densely sintered test body of the yttria-containing zirconia material, and
-    a biaxial flexural strength of at least 1200 MPa determined on a densely sintered test body of the yttria-containing zirconia material.

The fracture resistance may be determined by the indentation fracture (IF) method according to ISO14627, and particularly ISO14627:2012. Determination of the fracture resistance may be carried out by evaluating across cracks according to ISO14627, and particularly according to ISO14627:2012 (cf. "Method A" as described herein in section "Measuring methods"). Alternatively, the determination of the fracture resistance may be carried out by evaluating radial cracks using the Niihara equation for radial cracks (cf. "Method B" as described herein in section "Measuring methods").

The measuring method is carried out using a densely sintered test body of the yttria-containing zirconia material (e.g., densely sintered at a maximum sintering temperature between 1250 and 1350°C, like 1250°C or 1300°C). The densely sintered test body is typically obtained by cutting (e.g., milling) a section from the dental mill blank, densely sintering the section, and lapping and/or polishing the surface of the densely sintered section. The lapping and/or polishing is typically carried out according to DIN EN 843-1, and particularly DIN EN 843-1:2008-08. The section may be densely sintered at a maximum sintering temperature between 1250 and 1350°C, for example, at a maximum sintering temperature of 1250°C or 1300°C. The maximum sintering temperature may be hold for 2 hours. Further details for determining the fracture resistance of the yttria-containing zirconia material are described herein in section "Measuring methods".

The yttria-containing zirconia material may have a fracture resistance of at least 8.0 MPa*m$^{-1/2}$, and preferably at least 10.0 MPa*m$^{-1/2}$, like at least 12.0 MPa*m$^{-1/2}$, at least 13.0 MPa*m$^{-1/2}$, or at least 14.0 MPa*m$^{-1/2}$. The yttria-containing zirconia material may have a fracture resistance of at most 23.0 MPa*m$^{-1/2}$, at most 22.0 MPa*m$^{-1/2}$, at most 20.0 MPa*m$^{-1/2}$, or at most 19.0 MPa*m$^{-1/2}$. Thus, the yttria-containing zirconia material may have a fracture resistance in the range of 6.0 to 23.0 MPa*m$^{-1/2}$, preferably in the range of 8.0 to 22.0 MPa*m$^{-1/2}$, and more preferably in a range of 10.0 to 20.0 MPa*m$^{-1/2}$, like in a range of 11.0 to 19.5 MPa*m$^{-1/2}$, 12.0 to 19.0 MPa*m$^{-1/2}$, or 14.0 to 19.0 MPa*m$^{-1/2}$.

The yttria-containing zirconia material may have a fracture resistance, determined according to ISO14627 (particularly ISO14627:2012), "Method A" as described herein, of at least 11.0 MPa*m$^{-1/2}$, and preferably at least 13.0 MPa*m$^{-1/2}$, like at least 14.0 MPa*m$^{-1/2}$, at least 15.0 MPa*m$^{-1/2}$, or at least 15.5 MPa*m$^{-1/2}$. The yttria-containing zirconia material may have a fracture resistance, determined according to ISO14627 (particularly ISO14627:2012), "Method A" as described herein, of at most 23.0 MPa*m$^{-1/2}$, at most 22.0 MPa*m$^{-1/2}$, at most 20.0 MPa*m$^{-1/2}$, at most 19.0 MPa*m$^{-1/2}$. Thus, the yttria-containing zirconia material may have a fracture resistance, determined according to ISO14627 (particularly ISO14627:2012), "Method A" as described herein, in the range of 11.0 to 23.0 MPa*m$^{-1/2}$, preferably in the range of 13.0 to 22.0 MPa*m$^{-1/2}$, and more preferably in a range of 14.0 to 20.0 MPa*m$^{-1/2}$, like in a range of 15.0 to 19.5 MPa*m$^{-1/2}$ or 15.5 to 19.0 MPa*m$^{-1/2}$.

In one embodiment, the yttria-containing zirconia material has a fracture resistance, determined according to ISO14627 (particularly ISO14627:2012), "Method A" as described herein, of the range of 14.5 to 19.5 MPa*m$^{-1/2}$, like in a range of 15.0 to 18.5 MPa*m$^{-1/2}$, like in a range of 15.5 to 18.0 MPa*m$^{-1/2}$, and optionally wherein the yttria-containing zirconia material is an yttria-containing zirconia material according to "Embodiment P1" as described herein below. In one embodiment, the yttria-containing zirconia material has a fracture resistance, determined according to ISO14627 (particularly ISO14627:2012), "Method A" as described herein, of in the range of 16.0 to 20.5 MPa*m$^{-1/2}$, like in a range of 17.0 to 20.0 MPa*m$^{-1/2}$, like in a range of 17.5 to 19.5 MPa*m$^{-1/2}$, and optionally wherein the yttria-containing zirconia material is an yttria-containing zirconia material according to "Embodiment P2" as described herein below.

The yttria-containing zirconia material may have a fracture resistance, determined according to ISO14627 (particularly ISO14627:2012), "Method B" as described herein, of at least 8.0 MPa*m$^{-1/2}$, and preferably at least 10.0 MPa*m$^{-1/2}$, like at least 12.0 MPa*m$^{-1/2}$, at least 13.0 MPa*m$^{-1/2}$, or at least 14.0 MPa*m$^{-1/2}$. The yttria-containing zirconia material may have a fracture resistance, determined according to ISO14627 (particularly ISO14627:2012), "Method B" as described herein, of at most 19.0 MPa*m$^{-1/2}$, at most 18.0 MPa*m$^{-1/2}$, at most 17.5 MPa*m$^{-1/2}$, at most 17.0 MPa*m$^{-1/2}$. Thus, the yttria-containing zirconia material may have a fracture resistance, determined according to ISO14627 (particularly ISO14627:2012), "Method B" as described herein, in the range of 6.0 to 19.0 MPa*m$^{-1/2}$, preferably in the range of 8.0 to 19.0 MPa*m$^{-1/2}$, and more preferably in a range of 10.0 to 18.0 MPa*m$^{-1/2}$, like in a range of 11.0 to 17.5 MPa*m$^{-1/2}$, 12.0 to 17.0 MPa*m$^{-1/2}$, or 14.0 to 17.0 MPa*m$^{-1/2}$.

In one embodiment, the yttria-containing zirconia material has a fracture resistance, determined according to ISO14627 (particularly ISO14627:2012), "Method B" as described herein, of the range of 10.5 to 16.5 MPa*m$^{-1/2}$, like in a range of 11.0 to 16.0 MPa*m$^{-1/2}$, like in a range of 11.5 to 15.5 MPa*m$^{-1/2}$, and optionally wherein the yttria-containing zirconia material is an yttria-containing zirconia material according to "Embodiment P1" as described herein below. In one embodiment, the yttria-containing zirconia material has a fracture resistance, determined according to ISO14627 (particularly ISO14627:2012), "Method B" as described herein, of in the range of 13.5 to 18.5 MPa*m$^{-1/2}$, like in a range of 14.0 to 18.0 MPa*m$^{-1/2}$, like in a range of 14.5 to 17.5 MPa*m$^{-1/2}$, and optionally wherein the yttria-containing zirconia material is an yttria-containing zirconia material according to "Embodiment P2" as described herein below.

The biaxial flexural strength may be determined according to ISO 6872, and particularly ISO 6872:2008. The measuring method is carried out using a densely sintered test body of the yttria-containing zirconia material (e.g., densely sintered at a maximum sintering temperature between 1250 and 1350°C, like 1250°C or 1300°C). The densely sintered test body is typically obtained by cutting (e.g., milling) a section from the dental mill blank, densely sintering the section, and polishing the surface of the densely sintered section as described in ISO 6872, and particularly ISO 6872:2008. The section may be densely sintered at a maximum sintering temperature between 1250 and 1350°C, for example, at a maximum sintering temperature of 1250°C or 1300°C. The maximum sintering temperature may be hold for 2 hours. Further details for determining the biaxial flexural strength of the yttria-containing zirconia material are described herein in section "Measuring methods".

The yttria-containing zirconia material may have a biaxial flexural strength of at least 1300 MPa, and preferably at least 1350 MPa, like at least 1400 MPa or at least 1500 MPa. The yttria-containing zirconia material may have a biaxial flexural strength of at most 2200 MPa, at most 2150 MPa, at most 2000, or at most 1900 MPa. Thus, the yttria-containing zirconia material may have a biaxial flexural strength in the range of 1200 to 2200 MPa, preferably 1300 to 2200 MPa, and more preferably 1350 to 2200 MPa, like in a range of 1400 to 2150 MPa.

In one embodiment, the yttria-containing zirconia material has a biaxial flexural strength in the range of the range of 1350 to 1900 MPa, like in a range of 1400 to 1900 MPa, and optionally wherein the yttria-containing zirconia material is an yttria-containing zirconia material according to "Embodiment P1" as described herein below.

In one embodiment, the yttria-containing zirconia material has a biaxial flexural strength in the range of the range of 1700 to 2200 MPa, like in a range of 1750 to 2150 MPa, and optionally wherein the yttria-containing zirconia material is an yttria-containing zirconia material according to "Embodiment P2" as described herein below.

The yttria-containing zirconia material may be further characterized by its hardness in a densely sintered state. When it is referred herein to the "Vickers hardness VH" of the yttria-containing zirconia material, this refers to the hardness of the yttria-containing zirconia material, determined on a densely sintered test body of the yttria-containing zirconia material as described herein below. The Vickers hardness VH may be determined according to ISO14705, and particularly ISO14705:2008, using a test load of 196.1 N (HV20). The measuring method is carried out using a densely sintered test body of the yttria-containing zirconia material. The densely sintered test body is typically obtained by cutting (e.g., milling) a section from the dental mill blank, densely sintering the section, and lapping and/or polishing the surface of the densely sintered section. The lapping and/or polishing may be carried out according to DIN EN 843-1, and particularly DIN EN 843-1:2008-08. The section may be densely sintered at a maximum sintering temperature between 1250 and 1350°C, for example, at a maximum sintering temperature of 1250°C or 1300°C. The maximum sintering temperature may be hold for 2 hours. Further details for determining the Vickers hardness VH of the yttria-containing zirconia material are described herein in section "Measuring methods".

The yttria-containing zirconia material may be characterized by a Vickers hardness VH of at least 9 000 MPa, and preferably at least 10 000 MPa, like at least 11 000 MPa or at least 11 250 MPa. The yttria-containing zirconia material may have a Vickers hardness VH of at most 14 500 MPa, at most 13 500 MPa, or at most 12 750 MPa. The yttria-containing zirconia material may have a Vickers hardness VH in the range of 9 000 to 14 500 MPa, preferably at least 10 000 to 13 500 MPa, like in a range of 11 000 to 12 750 MPa or 11 250 to 12 750 MPa. In one embodiment, the yttria-containing zirconia material has a Vickers hardness VH in the range of 11 500 to 12 750 MPa, and optionally wherein the yttria-containing zirconia material is an yttria-containing zirconia material according to "Embodiment P1" as described herein below. In one embodiment, the yttria-containing zirconia material has a Vickers hardness VH in the range of 11 000 to 12 250 MPa, and optionally wherein the yttria-containing zirconia material is an yttria-containing zirconia material according to "Embodiment P2" as described herein below.

The yttria-containing zirconia material may have specific optical properties such as CIE L*a*b* color values, opacity or translucency. Unless explicitly stated otherwise, the optical properties of the yttria-containing zirconia material as described herein refer to the optical properties determined using a densely sintered test body of the yttria-containing zirconia material having a thickness of $1.00 \pm 0.05$ mm. The densely sintered test body is typically obtained by cutting (e.g., milling) a section from the dental mill blank, densely sintering the section, and polishing the surface of the densely sintered section. The section may be densely sintered at a maximum sintering temperature between 1250 and 1350°C, for example, at a maximum sintering temperature of 1250°C or 1300°C. The maximum sintering temperature may be held for 2 hours. Further details for determining the optical properties of the yttria-containing zirconia material are described herein in section "Measuring methods".

The yttria-containing zirconia material may have a contrast ratio of less than 90%, and preferably less than 88%, like less than 86%. The yttria-containing zirconia material may have a contrast ratio in the range of 70 to 90%, and preferably 72 to 88%, like in a range of 74 to 86%. In one embodiment, the yttria-containing zirconia material has a contrast ratio in the range of 82 to 88%, and optionally wherein the yttria-containing zirconia material is an yttria-containing zirconia material according to "Embodiment P2" as described herein below. In one embodiment, the yttria-containing zirconia material has a contrast ratio in the range of 72 to 81%, and optionally wherein the yttria-containing zirconia material is an yttria-containing zirconia material according to "Embodiment P1" as described herein below.

The contrast ratio may be determined according to BS 5612, and particularly BS 5612:1978. The contrast ratio relates to the ratio of illuminance (Y) of a material when placed on a black background (Yb) to the illuminance of the same material when placed over a white background (Yw) (CR = Yb/Yw). The contrast ratio can be used to characterize the translucency of a material, i.e. the light transmission of a material expressed as the ratio of transmitted to incident light intensity. A contrast ratio of close to 0% may indicate that a given material is almost fully transparent, while a contrast ratio of 100% may indicate that a material is fully opaque.

The yttria-containing zirconia material may have CIE L*a*b* color values of: L* in range of 76 to 96, a* in a range of (-2) to 6, b* in a range of 0 to 25.

CIE L*a*b* values can be measured according to DIN 6174. The measurements may be made against a background of, for example, L* = 93.1; a* = (-0.64); b* = 4.22. CIE L*a*b* values characterize a color of a material by a three-dimensional color space. An individual color L* is a measure of luminance lightness and it is represented on the vertical axis of the color space. The a* and b* coordinates, are a measure of chromaticity and are represented on the horizontal coordinates of the color space, with positive a* representing red, negative a* representing green, positive b* representing yellow and negative b* representing blue.

The CIE L*a*b* values of the yttria-containing zirconia material depend on the presence of coloring oxides in the material. The CIE L*a*b* values of the material are different in case the material comprises coloring oxides. The dental mill blank according to the present invention may be pre-colored or not pre-colored. This is also described in more detail in other parts of the present disclosure. In case the dental mill blank is a pre-colored dental mill blank, the yttria-containing zirconia material comprises coloring oxides in an amount and/or combination that is effective to impart a color (e.g., a color matching a natural color of tooth and/or matching a tooth color by VITA classical A1-D4® shade guide with VITA Bleached Shades manufactured by Vita Zahnfabrik or a similar dental shade guide system) to a dental restoration that is machined from the dental mill blank and then densely sintered. The color can be characterized by specific CIE L*a*b* values.

In one embodiment, the yttria-containing zirconia material has CIE L*a*b* color values of:

L* in range of 84 to 96, like in a range of 86 to 93,
a* in a range of (-2.0) to 1.0, like in a range of (-1.5) to 0.5,
b* in a range of 0.0 to 4.0, like in a range of 0.5 to 3.0.

The yttria-containing zirconia material is preferably further characterized by its chemical properties as described in the following section.

1.2 Chemical properties

One embodiment of the present invention provides a dental mill blank comprising an yttria-containing zirconia material, wherein the yttria-containing zirconia material comprises yttria in an amount of at most 4.0 wt.%, based on the total weight of the yttria-containing zirconia material.

The yttria-containing zirconia material is typically an yttria-stabilized zirconia material. The yttria-stabilized zirconia material may have a monoclinic ratio of crystal phases of less than 20%, less than 15% or less than 10%, based on the entire crystal phases of the material. The monoclinic ratio of crystal phases may be determined using quantitative X-ray diffractometry. The measuring method is known to the person of skill. The monoclinic ratio may be determined following DIN EN ISO 13356, (with reference to ASTM C1499). The yttria-containing zirconia material may have a theoretical density of about 6.1 $g/cm^3$, like about 6.11 $g/cm^3$.

The yttria-containing zirconia material comprises yttria. The yttria-containing zirconia material preferably comprises yttria in an amount of at most 4.0 wt.%, more preferably in a range of 1.5 to 4.0 wt.%, and even more preferably in a range of 2.0 to 3.8 wt.%, like in a range of 2.2 to 3.4 wt.%, based on the total weight of the yttria-containing zirconia material.

The yttria-containing zirconia material comprises zirconia. The yttria-containing zirconia material preferably comprises zirconia in an amount of at least 82.0 wt.%, more preferably at least 84.0 wt.%, even more preferably at least 86.0 wt.%, like at least 88.0 wt.%, at least 90.0 wt.%, at least 92.0 wt.% or at least 94.0 wt.%, based on the total weight of the yttria-containing zirconia material. The yttria-containing zirconia material may comprise zirconia in an amount in a range of 82.0 to 98.5 wt.%, preferably in a range of 84.0 to 98.5 wt.%, more preferably in a range of 86.0 to 98.0 wt.%, like in a range of 88.0 to 98.0 wt.%, in a range of 90.0 to 98.0 wt.%, in a range of 92.0 to 97.0 wt.% or in a range of 94.0 to 96.0 wt.%, based on the total weight of the yttria-containing zirconia material.

The yttria-containing zirconia material may comprise hafnia ($HfO_2$). The yttria-containing zirconia material may comprise hafnia in a weight ratio to zirconia in the range of 0:100 to 5:95, 1:99 to 4:96, 2:98 to 3:97, based on the total weight of the combined amount of hafnia and zirconia being present in the material. The yttria-containing zirconia material may comprise hafnia in an amount of at most 5.0 wt.%, preferably at most 4.0 wt.%, like in a range of 0.5 to 4.0 wt.%, 1.0 to 3.0 wt.% or 1.5 to 2.5 wt.%, based on the total weight of the yttria-containing zirconia material. The combined amount of zirconia and hafnia being present in the yttria-containing zirconia material may be in the range of from 92.5 to 98.5 wt.%, from 93.2 to 98.0 wt.%, or from 94.0 to 98.0 wt.%, based on the total weight of the yttria-containing zirconia material. A "combined amount of zirconia and hafnia" includes that an amount of hafnia may be 0 wt.% or that hafnia may be essentially absent from the yttria-containing zirconia material.

The yttria-containing zirconia material may comprise aluminum oxide ($Al_2O_3$). The material may comprise aluminum oxide in an amount of at most 2.0 wt.%, preferably in a range of 0.0 to 1.5 wt.%, more preferably in a range of 0.0 to 1.0 wt.%, and even more preferably in a range of 0.0 to 0.7 wt.%, like in a range of 0.0 to 0.5 wt.%, 0.1 to 0.7 wt.% or 0.1 to 0.5 wt.%, based on the total weight of the yttria-containing zirconia material. In one embodiment, the yttria-containing zirconia material comprises aluminum oxide in an amount of 0.1 to 0.4 wt.%, based on the total weight of the yttria-containing zirconia material. In one embodiment, the yttria-containing zirconia material comprises aluminum oxide in an amount of 0.4 to 0.7 wt.%, based on the total weight of the yttria-containing zirconia material.

The yttria-containing zirconia material may comprise oxides other than zirconia, yttria, hafnia and aluminum oxide. These other oxides may comprise coloring metal oxides and/or traces of metal oxides originating from the production process and/or raw material of the yttria-containing zirconia material. The amount of the other oxides may vary depending on whether or not the dental mill blank is a pre-colored dental mill blank. In case the dental mill blank is pre-colored, the amount and types of coloring metal oxides may depend on a color (e.g., a color matching a natural color of tooth and/or matching a tooth color by VITA classical A1-D4® shade guide with VITA Bleached Shades manufactured by Vita Zahnfabrik or a similar dental shade guide system) that is intended to be achieved for a dental restoration prepared using the dental mill blank. The dental mill blank may be pre-colored or may not be pre-colored.

The yttria-containing zirconia material may comprise oxides, other than zirconia, yttria, hafnia and aluminum oxide, in an amount of at most 5.0 wt.%, at most 4.0 wt.%, at most 3.0 wt.%, at most 2.0 wt.%, at most 1.0 wt.%, at most 0.5 wt.%, at most 0.3 wt.%, at most 0.3 wt.%. at most 0.1 wt.%, or at most 0.5 wt.%, based on the total weight of the porous zirconia ceramic material.

The yttria-containing zirconia material may comprise one or more coloring metal oxides. Suitable coloring metal oxides may be, but are not limited to, the oxides of Fe, Mn, Cr, Pr, Tb, Er, Yb, Ce, Co, Ni, Nd, Cu, Bi, and any mixture thereof. The one or more coloring metal oxides may comprise iron oxide, erbium oxide, chromium oxide, manganese oxide, terbium oxide, praseodymium oxide, or any combination thereof, and optionally one or more additional coloring metal oxides.

The yttria-containing zirconia material may comprise one or more coloring metal oxides in an amount of at least 0.02 wt.%, at least 0.1 wt.%, at least 0.2 wt.%, at most 2.0 wt.%, at most 1.5 wt.%, or at most 1.0 wt.%, at most 0.5 wt.%, based on the total weight of the yttria-containing zirconia material. The yttria-containing zirconia material may comprise one or more coloring metal oxides in an amount in the range of 0.02 to 2.0 wt.%, 0.1 to 1.5 wt.%, 0.2 to 1.0 wt.%, 0.1 to 1.0 wt.%, or 0.2 to 1.0 wt.%, based on the total weight of the yttria-containing zirconia material. It is also possible that the yttria-containing zirconia material comprises coloring metal oxides in an amount of less than 0.02 wt.%, based on the yttria-containing zirconia material. This is preferably the case when the dental mill blank is not pre-colored.

Traces of metal oxides originating from the production process and/or raw material of the yttria-containing zirconia material may be, but are not limited to, $Fe_2O_3$, $TiO_2$, $SiO_2$, $CaO$, or $Na_2O$.

The yttria-containing zirconia material may be characterized by a chemical composition as described in the following paragraphs. Unless explicitly stated otherwise, the weight amount of the indicated components are to be selected to add up to 100.0 wt.%. The indicated weight amounts of the components of the yttria-containing zirconia material are based on the total weight of the yttria-containing zirconia material. When the dental mill blank is composed of the yttria-containing zirconia material, the total weight of the yttria-containing material may be equated with the total weight of the dental mill blank.

The yttria-containing zirconia material may comprise

from 90.0 to 98.5 wt.%, preferably from 91.0 to 98.0 wt.%, like from 93.0 to 97.7 wt.%, of a combined amount of zirconia and hafnia,
from 1.5 to 4.0 wt.%, preferably from 2.0 to 3.8 wt.%, like from 2.2 to 3.4 wt.%, of yttria,
from 0.0 to 1.0 wt.%, preferably from 0.0 to 0.7 wt.%, like from 0.1 to 0.7 wt.% of aluminum oxide (e.g., from 0.1 to 0.4 wt.% or from 0.4 to 0.7 wt.%), and
from 0.0 to 5.0 wt.%, preferably from 0.0 to 4.5 wt.%, like from 0.0 to 3.0 wt.%, of other oxides, optionally comprising one or more coloring metal oxides optionally comprising iron oxide, erbium oxide, chromium oxide, manganese oxide, terbium oxide, praseodymium oxide, or any combination thereof.

The yttria-containing zirconia material may comprise

from 85.0 to 98.5 wt.%, preferably from 87.0 to 97.5 wt.%, like from 90.0 to 96.7 wt.%, of zirconia,
from 0.0 to 5.0 wt.%, preferably from 0.5 to 4.0 wt.%, like from 1.0 to 3.0 wt.%, of hafnia,
from 1.5 to 4.0 wt.%, preferably from 2.0 to 3.8 wt.%, like from 2.2 to 3.4 wt.%, of yttria,
from 0.0 to 1.0 wt.%, preferably from 0.0 to 0.7 wt.%, like from 0.1 to 0.7 wt.% of aluminum oxide (e.g., from 0.1 to 0.4 wt.% or from 0.4 to 0.7 wt.%), and
from 0.0 to 5.0 wt.%, preferably from 0.0 to 4.5 wt.%, like from 0.0 to 3.0 wt.%, of other oxides, optionally comprising one or more coloring metal oxides optionally comprising iron oxide, erbium oxide, chromium oxide, manganese oxide, terbium oxide, praseodymium oxide, or any combination thereof.

The yttria-containing zirconia material may comprise

from 90.0 to 98.5 wt.%, preferably from 91.0 to 98.0 wt.%, like from 93.0 to 97.7 wt.%, of a combined amount of zirconia and hafnia,
from 1.5 to 4.0 wt.%, preferably from 2.0 to 3.8 wt.%, like from 2.2 to 3.4 wt.%, of yttria,
from 0.0 to 1.0 wt.%, preferably from 0.0 to 0.7 wt.%, like from 0.1 to 0.7 wt.% of aluminum oxide (e.g., from 0.1 to 0.4 wt.% or from 0.4 to 0.7 wt.%),
from 0.0 to 2.0 wt.%, preferably from 0.0 to 1.5 wt.%, like from 0.0 to 1.0 wt.%, of one or more coloring metal oxides selected from the group consisting of iron oxide, erbium oxide, chromium oxide, manganese oxide, terbium oxide, praseodymium oxide, and any combination thereof,
from 0.0 to 3.0 wt.%, preferably from 0.0 to 2.5 wt.%, like from 0.0 to 2.0 wt.%, of other oxides (which may comprise additional coloring metal oxides which are not part of the defined group of coloring metal oxides).

The yttria-containing zirconia material may comprise

from 85.0 to 98.5 wt.%, preferably from 87.0 to 97.5 wt.%, like from 90.0 to 96.7 wt.%, of zirconia,
from 0.0 to 5.0 wt.%, preferably from 0.5 to 4.0 wt.%, like from 1.0 to 3.0 wt.%, of hafnia,
from 1.5 to 4.0 wt.%, preferably from 2.0 to 3.8 wt.%, like from 2.2 to 3.4 wt.%, of yttria,
from 0.0 to 1.0 wt.%, preferably from 0.0 to 0.7 wt.%, like from 0.1 to 0.7 wt.% of aluminum oxide (e.g., from 0.1 to 0.4 wt.% or from 0.4 to 0.7 wt.%), and
from 0.0 to 2.0 wt.%, preferably from 0.0 to 1.5 wt.%, like from 0.0 to 1.0 wt.%, of one or more coloring metal oxides selected from the group consisting of iron oxide, erbium oxide, chromium oxide, manganese oxide, terbium oxide, praseodymium oxide, and any combination thereof,
from 0.0 to 3.0 wt.%, preferably from 0.0 to 2.5 wt.%, like from 0.0 to 2.0 wt.%, of other oxides (which may comprise additional coloring metal oxides which are not part of the defined group of coloring metal oxides).

According to one embodiment, the yttria-containing zirconia material comprises

from 93.0 to 98.5 wt.%, preferably from 94.0 to 98.0 wt.%, like from 95.0 to 97.7 wt.%, of a combined amount of zirconia and hafnia,
from 1.5 to 4.0 wt.%, preferably from 2.0 to 3.8 wt.%, like from 2.2 to 3.4 wt.%, of yttria,
from 0.0 to 1.0 wt.%, preferably from 0.0 to 0.7 wt.%, like from 0.1 to 0.7 wt.% of aluminum oxide (e.g., from 0.1 to 0.4 wt.% or from 0.4 to 0.7 wt.%), and
from 0.0 to 2.0 wt.%, preferably from 0.0 to 1.5 wt.%, like from 0.0 to 1.0 wt.%, of other oxides, optionally comprising one or more coloring metal oxides optionally comprising iron oxide, erbium oxide, chromium oxide, manganese oxide, terbium oxide, praseodymium oxide, or any combination thereof.

According to one embodiment, the yttria-containing zirconia material comprises

from 88.0 to 98.5 wt.%, preferably from 90.0 to 97.5 wt.%, like from 92.0 to 96.7 wt.%, of zirconia,
from 0.0 to 5.0 wt.%, preferably from 0.5 to 4.0 wt.%, like from 1.0 to 3.0 wt.%, of hafnia,
from 1.5 to 4.0 wt.%, preferably from 2.0 to 3.8 wt.%, like from 2.2 to 3.4 wt.%, of yttria,
from 0.0 to 1.0 wt.%, preferably from 0.0 to 0.7 wt.%, like from 0.1 to 0.7 wt.% of aluminum oxide (e.g., from 0.1 to 0.4 wt.% or from 0.4 to 0.7 wt.%), and
from 0.0 to 2.0 wt.%, preferably from 0.0 to 1.5 wt.%, like from 0.0 to 1.0 wt.%, of other oxides, optionally comprising one or more coloring metal oxides optionally comprising iron oxide, erbium oxide, chromium oxide, manganese oxide, terbium oxide, praseodymium oxide, or any combination thereof.

According to one embodiment, the yttria-containing zirconia material comprises

from 93.0 to 98.5 wt.%, preferably from 94.0 to 98.0 wt.%, like from 95.0 to 97.7 wt.%, of a combined amount of zirconia and hafnia,
from 1.5 to 4.0 wt.%, preferably from 2.0 to 3.8 wt.%, like from 2.2 to 3.4 wt.%, of yttria,
from 0.0 to 1.0 wt.%, preferably from 0.0 to 0.7 wt.%, like from 0.1 to 0.7 wt.% of aluminum oxide (e.g., from 0.1 to 0.4 wt.% or from 0.4 to 0.7 wt.%),
from 0.0 to 2.0 wt.%, preferably from 0.0 to 1.5 wt.%, like from 0.0 to 1.0 wt.%, of one or more coloring metal oxides, optionally comprising iron oxide, erbium oxide, chromium oxide, manganese oxide, terbium oxide, praseodymium oxide, or any combination thereof, and
optionally less than 0.2 wt.% of other oxides (e.g., $TiO_2$, $SiO_2$, CaO, and/or $Na_2O$).

According to one embodiment, the yttria-containing zirconia material comprises

from 88.0 to 98.5 wt.%, preferably from 90.0 to 97.5 wt.%, like from 92.0 to 96.7 wt.%, of zirconia,
from 0.0 to 5.0 wt.%, preferably from 0.5 to 4.0 wt.%, like from 1.0 to 3.0 wt.%, of hafnia,
from 1.5 to 4.0 wt.%, preferably from 2.0 to 3.8 wt.%, like from 2.2 to 3.4 wt.%, of yttria,
from 0.0 to 1.0 wt.%, preferably from 0.0 to 0.7 wt.%, like from 0.1 to 0.7 wt.% of aluminum oxide (e.g., from 0.1 to 0.4 wt.% or from 0.4 to 0.7 wt.%),
from 0.0 to 2.0 wt.%, preferably from 0.0 to 1.5 wt.%, like from 0.0 to 1.0 wt.%, of one or more coloring metal oxides, optionally comprising iron oxide, erbium oxide, chromium oxide, manganese oxide, terbium oxide, praseodymium oxide, or any combination thereof, and
optionally less than 0.2 wt.% of other oxides (e.g., $TiO_2$, $SiO_2$, CaO, and/or $Na_2O$).

According to one embodiment, the yttria-containing zirconia material comprises

from 95.0 to 98.5 wt.%, preferably from 95.5 to 98.0 wt.%, like from 96.0 to 97.7 wt.%, of a combined amount of zirconia and hafnia,
from 1.5 to 4.0 wt.%, preferably from 2.0 to 3.8 wt.%, like from 2.2 to 3.4 wt.%, of yttria,
from 0.0 to 1.0 wt.%, preferably from 0.0 to 0.7 wt.%, like from 0.1 to 0.7 wt.% of aluminum oxide (e.g., from 0.1 to 0.4 wt.% or from 0.4 to 0.7 wt.%), and
optionally less than 0.2 wt.% of other oxides (e.g., $Fe_2O_3$, $TiO_2$, $SiO_2$, CaO, and/or $Na_2O$).

According to one embodiment, the yttria-containing zirconia material comprises

from 90.0 to 98.5 wt.%, preferably from 91.5 to 97.5 wt.%, like from 93.0 to 96.7 wt.%, of zirconia,
from 0.0 to 5.0 wt.%, preferably from 0.5 to 4.0 wt.%, like from 1.0 to 3.0 wt.%, of hafnia,
from 1.5 to 4.0 wt.%, preferably from 2.0 to 3.8 wt.%, like from 2.2 to 3.4 wt.%, of yttria,
from 0.0 to 1.0 wt.%, preferably from 0.0 to 0.7 wt.%, like from 0.1 to 0.7 wt.% of aluminum oxide, and
optionally less than 0.2 wt.% of other oxides (e.g., $Fe_2O_3$, $TiO_2$, $SiO_2$, CaO, and/or $Na_2O$).

According to one embodiment (also referred to herein as "Embodiment P1"), the yttria-containing zirconia material comprises

from 95.8 to 97.9 wt.%, like from 96.3 to 97.6 wt.%, of a combined amount of zirconia and hafnia,
preferably from 2.0 to 3.8 wt.%, like from 2.2 to 3.4 wt.%, of yttria,
preferably from 0.1 to 0.4 wt.%, like from 0.2 to 0.3 wt.% of aluminum oxide, and
optionally less than 0.1 wt.% of other oxides (e.g., $Fe_2O_3$, $TiO_2$, $SiO_2$, CaO, and/or $Na_2O$).

According to one embodiment (also referred to herein as "Embodiment P2"), the yttria-containing zirconia material comprises

from 91.5 to 97.1 wt.%, like from 93.0 to 96.4 wt.%, of zirconia,
from 0.5 to 4.0 wt.%, like from 1.0 to 3.0 wt.%, of hafnia,
from 2.0 to 3.8 wt.%, like from 2.2 to 3.4 wt.%, of yttria,
from 0.4 to 0.7 wt.%, like 0.4 to 0.6 wt.%, of aluminum oxide, and
optionally less than 0.2 wt.% of other oxides (e.g., $TiO_2$, $SiO_2$, CaO, and/or $Na_2O$).

**2. Structure of the dental mill blank**

The dental mill blank is porous. The dental mill blank is typically open-porous or may have an open-porous structure. The density of the dental mill blank, relative to its theoretical density, may be at least 45%, like in a range of 45 to 70%, 45 to 60%, or 45 to 55%. The theoretical density of the dental mill blank may be about 6.1 g/cm$^3$, like about 6.11 g/cm$^3$. The density of the dental mill blank may be in a range of 2.5 to 4.2 g/cm$^3$, 2.6 to 3.8 g/cm$^3$, or 2.7 to 3.4 g/cm$^3$.
The dental mill blank may comprise the yttria-containing zirconia material in an amount of at least 98 wt.%, at least 99 wt.%, or at least 99.8 wt.%, based on the total weight of the dental mill blank. The dental mill blank may be composed of, essentially consist of or consist of the yttria-containing zirconia material. In one embodiment of the present invention, the dental mill blank is composed of the yttria-containing zirconia material.
Alternatively, the dental mill blank may comprise the yttria-containing zirconia material in form of a subsection of the dental mill blank. In such embodiment, the subsection is composed of the yttria-containing zirconia material. When the dental mill blank comprise the yttria-containing zirconia material in form of a subsection, the test bodies as described herein are prepared from the subsection. The subsection of the dental mill blank may be useful to prepare a subsection of a dental restoration precursor. The remaining part of the dental mill blank may be composed of other materials to prepare other subsections of a dental restoration precursor.
The dental mill blank may be characterized by its hardness, like its white hardness. The "white hardness" as used herein refers to the hardness of the porous dental mill blank in a pre-sintered state. Thus, when the white hardness is defined herein, this refers to a porous, pre-sintered dental mill blank according to an embodiment of the invention. The white hardness of a dental mill blank is relevant for its machinability and/or millability. The white hardness may be determined according to ISO14705, and particularly ISO14705:2008, using a test load of 24.5 N (HV2.5). The measuring method is carried out using a test body which is obtained by cutting (e.g., milling) a section from the dental mill blank. The test body is not subjected to a densely sintering but is tested in its porous state. Further details for determining the white hardness of the dental mill blank are described herein in section "Measuring methods".

The dental mill blank may have a white hardness of at least 250 MPa, and preferably at least 300 MPa, like at least 350 MPa or at least 400 MPa. The dental mill blank may have a white hardness of at most 1 000 MPa, at most 900 MPa, or at most 850 MPa. The dental mill blank may have a white hardness in the range of 250 to 1 000 MPa, preferably in a range 300 to 900 MPa, like in a range of 350 to 850 MPa or in a range 400 to 850 MPa. In one embodiment, the dental mill blank has a white hardness in the range of 250 to 550 MPa, like in a range 300 to 550 MPa, like in a range of 350 to 500 MPa. In an alternative embodiment, the dental mill blank has a white hardness in the range of 600 to 1 000 MPa, like in a range 650 to 950 MPa, like in a range of 700 to 900 MPa.

The dental mill blank is not particularly limited as regards its shape or its dimensions as long as it is suitable for use for preparing a dental restoration precursor (e.g., using a CAD/CAM process). The dental mill blank may have, but is not limited to, the form of rectangular block, a disc, a cylinder, a dental preform (e.g., an abutment preform or a tooth sector), a cone, a cone segment, a pyramid, or a pyramid segment. In one embodiment, the dental mill blank has the form of a disc, a cylinder or a rectangular block. For example, the dental mill blank may be, but is not limited to, a disc with a height in the range of 8 to 30 mm (e.g., about 10 mm, 12 mm, 14 mm, 16 mm, 18 mm, 20 mm, 25 mm), like in the range of 10 to 20 mm, and a diameter in the range of 40 to 150 mm. For example, the dental mill blank may have a diameter of about 40 mm and a height in a range of 10 to 20 mm. Other dimensions are also possible.

The dental mill blank is typically a pre-sintered dental mill blank. The dental mill blank may be obtained by pre-sintering a green body of the dental mill blank at a maximum pre-sintering temperature of at least 600°C, at least 650°C, or at least 675°C, like in a range of 600 to 800°C, in a range of 650 to 750°C or in a range of 675 to 725°C. The maximum pre-sintering temperature may be held for a time period in the range of 1 to 5 hours, like in the range of 2 to 3 hours. The green body may be a compacted green body that is obtainable using a compacting pressure (e.g., applied by cold isostatic pressing) in the range of 200 to 400 MPa, like in a range of 300 to 400 MPa.

In one embodiment, the dental mill blank is obtainable by the process for preparing a dental mill blank according to the present invention. The process is described in further detail in the following section.

## II. Process for preparing the dental mill blank

One aspect of the present invention provides a process for preparing a dental mill blank according to an embodiment of the present invention. The process comprises the steps of (carried out in that order):

- providing a zirconia powder,
- optionally treating the zirconia powder with one or more colorants,
- compacting the zirconia powder to provide a green body,
- pre-sintering the green body to provide a dental mill blank, and
- optionally pre-coloring the dental mill blank.

The process comprises the step of providing a zirconia powder. The zirconia powder may be characterized by its chemical composition as described in the following. Unless explicitly stated otherwise, the indicated weight amounts are to be selected to add up to 100.0 wt.% and are based on the total weight of the zirconia powder.

The zirconia powder may comprise

from 95.0 to 98.5 wt.%, preferably from 95.5 to 98.0 wt.%, like from 96.0 to 97.7 wt.%, of a combined amount of zirconia and hafnia,
from 1.5 to 4.0 wt.%, preferably from 2.0 to 3.8 wt.%, like from 2.2 to 3.4 wt.%, of yttria,
from 0.0 to 1.0 wt.%, preferably from 0.0 to 0.7 wt.%, like from 0.1 to 0.7 wt.% of aluminum oxide (e.g., from 0.1 to 0.4 wt.% or from 0.4 to 0.7 wt.%), and
from 0.0 to 0.3 wt.%, preferably from 0.0 to 0.1 wt.%, like from 0.0 to 0.05 wt.%, of other oxides (e.g., $Fe_2O_3$, $TiO_2$, $SiO_2$, CaO, and/or $Na_2O$).

The zirconia powder may comprise

from 90.0 to 98.5 wt.%, preferably from 91.5 to 97.5 wt.%, like from 93.0 to 96.7 wt.%, of zirconia,
from 0.0 to 5.0 wt.%, preferably from 0.5 to 4.0 wt.%, like from 1.0 to 3.0 wt.%, of hafnia,
from 1.5 to 4.0 wt.%, preferably from 2.0 to 3.8 wt.%, like from 2.2 to 3.4 wt.%, of yttria,
from 0.0 to 1.0 wt.%, preferably from 0.0 to 0.7 wt.%, like from 0.1 to 0.7 wt.% of aluminum oxide (e.g., from 0.1 to 0.4 wt.% or from 0.4 to 0.7 wt.%), and
from 0.0 to 0.3 wt.%, preferably from 0.0 to 0.1 wt.%, like from 0.0 to 0.05 wt.%, of other oxides (e.g., $Fe_2O_3$, $TiO_2$, $SiO_2$, CaO, or $Na_2O$).

In one embodiment, the zirconia powder comprises

from 95.5 to 98.0 wt.%, like from 96.0 to 97.7 wt.%, of a combined amount of zirconia and hafnia,
from 2.0 to 3.8 wt.%, like from 2.2 to 3.4 wt.%, of yttria,
from 0.1 to 0.4 wt.%, like from 0.2 to 0.3 wt.%, of aluminum oxide, and
from 0.0 to 0.1 wt.%, like from 0.0 to 0.05 wt.%, of other oxides (e.g., $Fe_2O_3$, $TiO_2$, $SiO_2$, CaO, or $Na_2O$).

In one embodiment, the zirconia powder comprises

from 91.5 to 97.5 wt.%, like from 93.0 to 96.7 wt.%, of zirconia,
0.5 to 4.0 wt.%, like from 1.0 to 3.0 wt.%, of hafnia,
from 2.0 to 3.8 wt.%, like from 2.2 to 3.4 wt.%, of yttria,
0.4 to 0.7 wt.%, like from 0.5 to 0.6 wt.%, of aluminum oxide, and
from 0.0 to 0.1 wt.%, like from 0.0 to 0.05 wt.%, of other oxides (e.g., $Fe_2O_3$, $TiO_2$, $SiO_2$, CaO, or $Na_2O$).

A skilled person knows how to obtain the zirconia powders described herein. The zirconia powder may be obtained as a commercial product, e.g., from Tosoh Corporation, Japan. The zirconia powder may be provided in combination with a binder, in particular an organic binder. However, it is also possible to provide the zirconia powder without an organic binder or a binder. The zirconia powder may also be provided in form of an aqueous suspension.

The process may comprise a step of treating the zirconia powder with one or more colorants. Treating the zirconia powder with one or more colorants is optional. In one embodiment, the process does not comprise a step of treating the zirconia powder with one or more colorants and/or one or more sintering additives before a compacting step.

The process may comprise treating the zirconia powder with one or more colorants. The colorants may be added in form of a solution, like an aqueous solution. Such a treatment step is known in the art and is described in, e.g., EP2707342 B1. Suitable colorants may be, but are not limited to, polyvalent ions of 3d- and/of 4f-elements in different valence states, like e.g. $Fe^{3+}$, $Mn^{2+}$, $Pr^{3+}$, $Tb^{3+}$, $Cr^{3+}$, and $Er^{3+}$, like salts of those compounds (e.g., nitrate salts). When the process comprises the treating of the zirconia powder with one or more colorants before a compacting step, it may be dispensed with coloring the dental mill blank towards the end of the process and as described further below. However, it is also possible to combine the two steps, if needed.

The process further comprises a step of compacting the zirconia powder to provide a green body. The optionally treated zirconia powder may be charged in a mold followed by a compacting of the powder. The compacting may be a pressing, like an uniaxial pressing, a cold isostatic pressing, or a combination of both. Thus, the compacting may comprise two or more pressing steps. In one embodiment, the compacting comprises an uniaxial pressing, followed by a cold isostatic pressing. In case the zirconia powder is provided in form of an aqueous suspension, the compacting may be carried out by a suitable wet-pressing technique, like silt casting and/or pressure filtration. The compacting, like the uniaxial pressing, the cold isostatic pressing, or the combination of both, may be carried out using pressures in the range of 200 to 400 MPa, like in a range of 300 to 400 MPa. In one embodiment, the compacting comprises an uniaxial pressing using a pressure in a range of 10 to 150 MPa, followed by a cold isostatic pressing using a pressure in a range of 300 to 400 MPa. The green body may have a density in a range of 2.5 to 4.2 $g/cm^3$, 2.6 to 3.8 $g/cm^3$, or 2.7 to 3.4 $g/cm^3$.

The process further comprises the pre-sintering of the green body to provide a dental mill blank. The pre-sintering may comprise a debinding of the green body. However, it is also possible to carry out a thermal debinding, e.g., in an air furnace, before pre-sintering. The pre-sintering may be carried out at a maximum pre-sintering temperature of at least 600°C, at least 650°C, or at least 675°C, like in a range of 600 to 800°C, in the range of 650 to 750°C, or in the range of 675 to 725°C. The maximum pre-sintering temperature may be held for a time period in the range of 1 to 5 hours, like in the range of 2 to 3 hours. In one embodiment, the pre-sintering is carried out at a maximum pre-sintering temperature in a range of 600 to 800°C, in the range of 650 to 750°C, or in the range of 675 to 725°C, wherein the maximum pre-sintering temperature is held for a time period in the range of 1 to 5 hours, like in the range of 2 to 3 hours.

The process may further comprise the pre-coloring of the dental mill blank. The pre-coloring may be carried out by treating, e.g., soaking or painting, the dental mill blank with one or more colorants. The one or more colorants may be provided in form of a solution, like an aqueous solution. Suitable colorants may be, but are not limited to, polyvalent ions of 3d- and/of 4f-elements in different valence states, like e.g. $Fe^{3+}$, $Mn^{2+}$, $Pr^{3+}$, $Tb^{3+}$, $Cr^{3+}$, and $Er^{3+}$, like salts of those compounds (e.g., nitrate salts). When the process comprises the pre-coloring of the dental mill blank after pre-sintering, it may be dispensed with treating the zirconia powder with colorants in a previous process step. However, both steps may also be combined.

The process may comprise one or more additional process steps which are typical in the art, and which may be carried out before, between or after the process steps described herein. Additional steps may be, but are not limited to, adjusting the particle size distribution of the zirconia powder (e.g., by sieving) and/or preparing the surface of the green body and/or the dental mill blank (e.g., by grinding, lapping or polishing).

### III. Dental restoration and a process for preparing the same

The dental mill blank according to the present invention is useful for preparing a dental restoration. One aspect of the present invention relates to the use of the dental mill blank according to the present invention for preparing a dental restoration. One aspect relates to a process for preparing a dental restoration using a dental mill blank according to the present invention.

One aspect of the present invention provides a process for preparing a dental restoration. The process comprises the steps of (carried out in that order):

- machining a dental mill blank according to an embodiment of the present invention to provide a dental restoration precursor;
- optionally surface-treating the dental restoration precursor;
- sintering the dental restoration precursor to provide a dental restoration.

The process comprises the step of machining a dental mill blank according to an embodiment of the present invention to provide a dental restoration precursor. The machining of the dental mill blank may be carried out by any conventional process for machining a dental mill blank, and typically by a CAD/CAM process. Such processes are known in the art. The machining may include, but is not limited to, cutting, drilling and/or grinding the dental mill blank. The dental restoration precursor typically has an open-porous structure.

The process optionally comprises the surface-treating of the dental restoration precursor, e.g., by polishing and/or coloring, or any other known step for modifying the surface of a dental restoration precursor. The surface-treating may comprise a manually surface-treating of the dental restoration precursor (e.g., manually surface polishing using a rotating dental polishing tool). The surface-treating may comprise coloring the dental restoration precursor, e.g., by an infiltration technique (e.g., brush infiltration technique) using a coloring liquid. Such a coloring step is known in the art and suitable coloring liquids are commercially available.

The process comprises the step of sintering the dental restoration precursor to provide a dental restoration. The sintering may be carried out by a sintering process for sintering dental restoration precursor, and particularly zirconia-based dental restoration precursor, as known in the art. Suitable sintering furnaces are known to the skilled person.

The sintering may be carried out at a maximum sintering temperature of at most 1350°C, like in a range of 1200 to 1350°C. It has been found that a dental restoration with good mechanical and optical properties may be obtained using a comparatively low maximum sintering temperature of at most 1350°C, like in a range of 1200 to 1350°C. This is advantageous in terms of energy consumption and may also shorten the overall sintering time.

The process may further comprise additional steps after the sintering step. For example, the process may comprise the coloring and/or glazing of the dental restoration as known in the art.

Another aspect of the present invention provides a dental restoration comprising (or essentially consisting of) an yttria-containing zirconia ceramic. The dental restoration may have a desirable color. The dental restoration may have a color that matches a shade of by the VITA classical A1-D4® shade guide with VITA Bleached Shades manufactured by Vita Zahnfabrik. A shade may be, but is not limited to, A1, A2, A3, A3.5, A4, B1, B2, B3, B4, C1, C2, C3, C4, D1, D2, D3, D4, BL1 or BL2. The yttria-containing zirconia ceramic may have specific optical properties such as CIE L*a*b* color values, opacity or translucency. The optical properties can be determined according to BS 5612 (e.g., for determining contrast ratio) and/or according to DIN 6174 (e.g. for determining CIE L*a*b* color values) using specimen having a thickness of $1.00 \pm 0.05$ mm. The yttria-containing zirconia ceramic may have CIE L*a*b* color values of: L* in range of 76 to 96, a* in a range of (-2) to 6, b* in a range of 0 to 25. The yttria-containing zirconia ceramic may have a contrast ratio in the range of 70 to 90%, and preferably 72 to 88%, like in a range of 74 to 86%.

The yttria-containing zirconia ceramic preferably comprises yttria in an amount of at most 4.0 wt.%, more preferably in an range of 1.5 to 4.0 wt.%, and even more preferably in a range of 2.0 to 3.8 wt.%, like in a range of 2.2 to 3.4 wt.%, based on the total weight of the yttria-containing zirconia ceramic. The yttria-containing zirconia ceramic preferably comprises zirconia in an amount in a range of 82.0 to 98.5 wt.%, preferably in a range of 84.0 to 98.5 wt.%, more preferably in a range of 86.0 to 98.0 wt.%, like in a range of 88.0 to 98.0 wt.%, in a range of 90.0 to 98.0 wt.%, in a range of 92.0 to 97.0 wt.% or in a range of 94.0 to 96.0 wt.%, based on the total weight of the yttria-containing zirconia ceramic. The yttria-containing zirconia ceramic may comprise hafnia in a weight ratio to zirconia in the range of 0:100 to 5:95, 1:99 to 4:96, 2:98 to 3:97, based on the total weight of the combined amount of hafnia and zirconia in the yttria-containing zirconia ceramic. The yttria-containing zirconia ceramic may comprise aluminum oxide in an amount of at most 2.0 wt.%, preferably in a range of 0.0 to 1.5 wt.%, more preferably in a range of 0.0 to 1.0 wt.%, and even more preferably in a range of 0.0 to 0.7 wt.%, like in a range of 0.0 to 0.5 wt.%, 0.1 to 0.7 wt.% or 0.1 to 0.5 wt.%, based on the total weight of the yttria-containing zirconia ceramic. In one embodiment, the yttria-containing zirconia ceramic comprises aluminum oxide in an amount of 0.1 to 0.4 wt.%, based on the total weight of the yttria-containing zirconia ceramic. In one embodiment, the yttria-containing zirconia ceramic comprises aluminum oxide in an amount of 0.4 to 0.7 wt.%, based on the total weight of the yttria-containing zirconia ceramic.

The yttria-containing zirconia ceramic typically comprises coloring metal oxides. Suitable coloring metal oxides may be, but are not limited to, the oxides of Fe, Mn, Cr, Pr, Tb, Er, Yb, Ce, Co, Ni, Nd, Cu, Bi, and any mixture thereof. The one or more coloring metal oxides may comprise iron oxide, erbium oxide, chromium oxide, manganese oxide, terbium oxide, praseodymium oxide, or any combination thereof, and optionally one or more additional coloring metal oxides. The yttria-containing zirconia ceramic may comprise one or more coloring metal oxides in an amount in the range of 0.02 to 2.0 wt.%, 0.1 to 1.5 wt.%, 0.2 to 1.0 wt.%, 0.1 to 1.0 wt.%, or 0.2 to 1.0 wt.%, based on the total weight of the yttria-containing zirconia ceramic.

The yttria-containing zirconia ceramic may comprise from 90.0 to 98.5 wt.%, preferably from 91.0 to 98.0 wt.% (e.g., from 93.0 to 97.7 wt.%), of a combined amount of zirconia and hafnia, from 1.5 to 4.0 wt.%, preferably from 2.0 to 3.8 wt.%, (e.g., from 2.2 to 3.4 wt.%, of yttria), from 0.0 to 1.0 wt.%, preferably from 0.0 to 0.7 wt.% (e.g., from 0.1 to 0.4 wt.% or from 0.4 to 0.7 wt.%), and from 0.0 to 5.0 wt.%, preferably from 0.0 to 4.5 wt.% (e.g., from 0.0 to 3.0 wt.%), of other oxides, optionally comprising one or more coloring metal oxides optionally comprising iron oxide, erbium oxide, chromium oxide, manganese oxide, terbium oxide, praseodymium oxide, or any combination thereof.

The yttria-containing zirconia ceramic may comprise from 85.0 to 98.5 wt.%, preferably from 87.0 to 97.5 wt.% (e.g., from 90.0 to 96.7 wt.%), of zirconia, from 0.0 to 5.0 wt.%, preferably from 0.5 to 4.0 wt.% (e.g., from 1.0 to 3.0 wt.%), of hafnia, from 1.5 to 4.0 wt.%, preferably from 2.0 to 3.8 wt.% (e.g., from 2.2 to 3.4 wt.%), of yttria, from 0.0 to 1.0 wt.%, preferably from 0.0 to 0.7 wt.% of aluminum oxide (e.g., from 0.1 to 0.4 wt.% or from 0.4 to 0.7 wt.%), and from 0.0 to 5.0 wt.%, preferably from 0.0 to 4.5 wt.% (e.g., from 0.0 to 3.0 wt.%), of other oxides, optionally comprising one or more coloring metal oxides optionally comprising iron oxide, erbium oxide, chromium oxide, manganese oxide, terbium oxide, praseodymium oxide, or any combination thereof. The weight amount of the indicated components are to be selected to add up to 100.0 wt.%. The indicated weight amounts of the components of the yttria-containing zirconia ceramic are based on the total weight of the yttria-containing zirconia ceramic.

In further embodiments, the yttria-containing zirconia ceramic may be characterized by any one of the chemical compositions as described herein above for the yttria-containing zirconia material being present in the inventive dental mill blank.

The dental restoration may be, but is not limited to, a crown, a partial crown, an abutment, an abutment crown, an inlay, an onlay, a veneer, a shell, or a multi-unit framework, or a bridge (e.g., 2-, 3- or 4- unit bridge) an implant bridge, and the like.

One embodiment of the present invention provides a dental restoration that is obtainable by the process for preparing a dental restoration according to an embodiment of the present invention. The dental restoration may be further characterized as defined herein above.

### IV. Use of a zirconia powder

Another aspect of the present invention provides a use of a zirconia powder for preparing a dental mill blank, wherein the zirconia powder comprises

from 95.0 to 98.5 wt.%, preferably from 95.5 to 98.0 wt.%, like from 96.0 to 97.7 wt.%, of a combined amount of zirconia and hafnia,
from 1.5 to 4.0 wt.%, preferably from 2.0 to 3.8 wt.%, like from 2.2 to 3.4 wt.%, of yttria,
from 0.0 to 1.0 wt.%, preferably from 0.0 to 0.7 wt.%, like from 0.1 to 0.7 wt.% of aluminum oxide (e.g., from 0.1 to 0.4 wt.% or from 0.4 to 0.7 wt.%), and
from 0.0 to 0.3 wt.%, preferably from 0.0 to 0.1 wt.%, like from 0.0 to 0.05 wt.%, of other oxides (e.g., $Fe_2O_3$, $TiO_2$, $SiO_2$, CaO, or $Na_2O$). The weight amounts are selected to add up to 100.0 wt.% and are based on the total weight of the zirconia powder.

The zirconia powder may comprise

from 90.0 to 98.5 wt.%, preferably from 91.5 to 97.5 wt.%, like from 93.0 to 96.7 wt.%, of zirconia,
from 0.0 to 5.0 wt.%, preferably from 0.5 to 4.0 wt.%, like from 1.0 to 3.0 wt.%, of hafnia,
from 1.5 to 4.0 wt.%, preferably from 2.0 to 3.8 wt.%, like from 2.2 to 3.4 wt.%, of yttria,
from 0.0 to 1.0 wt.%, preferably from 0.0 to 0.7 wt.%, like from 0.1 to 0.7 wt.% of aluminum oxide (e.g., from 0.1 to 0.4 wt.% or from 0.4 to 0.7 wt.%), and
from 0.0 to 0.3 wt.%, preferably from 0.0 to 0.1 wt.%, like from 0.0 to 0.05 wt.%, of other oxides (e.g., $Fe_2O_3$, $TiO_2$, $SiO_2$, CaO, or $Na_2O$).

In one embodiment, the zirconia powder comprises

from 95.5 to 98.0 wt.%, like from 96.0 to 97.7 wt.%, of a combined amount of zirconia and hafnia,

from 2.0 to 3.8 wt.%, like from 2.2 to 3.4 wt.%, of yttria,

from 0.1 to 0.4 wt.%, like from 0.2 to 0.3 wt.% of aluminum oxide, and

from 0.0 to 0.1 wt.%, like from 0.0 to 0.05 wt.%, of other oxides (e.g., $Fe_2O_3$, $TiO_2$, $SiO_2$, CaO, or $Na_2O$).

In one embodiment, the zirconia powder comprises

from 91.5 to 97.5 wt.%, like from 93.0 to 96.7 wt.%, of zirconia,

0.5 to 4.0 wt.%, like from 1.0 to 3.0 wt.%, of hafnia,

from 2.0 to 3.8 wt.%, like from 2.2 to 3.4 wt.%, of yttria,

0.4 to 0.7 wt.%, like from 0.5 to 0.6 wt.% of aluminum oxide, and

from 0.0 to 0.1 wt.%, like from 0.0 to 0.05 wt.%, of other oxides (e.g., $Fe_2O_3$, $TiO_2$, $SiO_2$, CaO, or $Na_2O$).

The zirconia powder may be used to prepare a dental mill blank according to an embodiment of the present invention.

In the following, the present invention is described by way of specific examples which shall not be construed as limiting the invention in any way.

## V. Example section

### 1. Measuring methods

#### 1.1 Biaxial flexural strength

Biaxial flexural strength was determined according to ISO 6872:2008. Each one of the test bodies was prepared by milling a section from the dental mill blank, followed by densely sintering the section in a sinter oven, and surface-polishing of the sintered section to provide the test body. The sintering was carried out in a commercial sinter oven (Programat S1 1600) using the sinter program as described in table 1 with a maximum sintering temperature T(max) of 1250°C or 1300°C. The sintered section had the following dimensions: 13 mm (d), 1.2 mm (h) with a 0.12 mm chamfer. The surface polishing was done in two steps: (1) polishing with diamond disc (35 μm grain size; 20 N) until surface is free of milling grooves; (2) polishing with cloth using diamond suspension (15 μm grain size; 10 N; 3 x 6 min).

Table 1: sintering program

| Step | T(start) [°C] | T(end) [°C] | Rate [K/min] | Holding time of T(end) [min] |
|---|---|---|---|---|
| S1 | 25 | 900 | 10 | 30 |
| S2 | 900 | T(max) | 3 | 120 |
| S3 | T(max) | 900 | -10 | - |
| S4 | 900 | 300 | -8 | - |
| Opening of sinter oven (15 min) | | | | |

#### 1.2 Fracture resistance

Fracture resistance was determined by the indentation fracture (IF) method according to ISO14627:2012 with a test load of 196.1 N (HV20). Each one of the test bodies was prepared by milling a section from the dental mill blank, followed by densely sintering the section in a sinter oven, and surface preparation of the sintered section to provide the test body. The sintering was carried out in a commercial sinter oven (Programat S1 1600) using the sinter program as described in table 1 with a maximum sintering temperature T(max) of 1250°C or 1300°C. The surface of the sintered section was lapped /polished according to DIN EN 843-1:2008-08. The test body had a thickness of 2.0 mm.

Two methods were used to calculate fracture resistance based on different analysis of the cracks:

- Method A: across cracks according to ISO14627:2012
- Method B: radial cracks according to Niihara's equation for radial cracks (see equation (I) below):

$$KIC = 0.018 * H * \sqrt{a} * \left(\frac{E}{H}\right)^{0.4} * \left(\frac{c}{a} - 1\right)^{-\frac{1}{2}} \quad (I)$$

Analysis of radial cracks according to Niihara's equation (I) is known to the skilled person, e.g., from pages 34 to 37 of D. Munz, T. Fett; Ceramics, "Mechanical Properties, Failure Behaviour, Materials Selection", 1999, ISBN 3-540-65376-7, Springer Verlag Berlin Heidelberg.

1.3 White hardness

The hardness of the dental mill blank in the pre-sintered state (white hardness) was determined according to ISO14705:2008 with a test load of 24.5 N (HV2.5). Each one of the test bodies was prepared by milling a section from the dental mill blank. The section was not subjected to a subsequent sintering step for densely sintering.

1.4 Vickers hardness

The Vickers hardness was determined according to ISO14705:2008 with test load of 196.1 N (HV20). Each one of the test bodies was prepared by milling a section from the dental mill blank, followed by densely sintering the section in a sinter oven, and surface preparation of the sintered section to provide the test body. The sintering was carried out in a commercial sinter oven (Programat S1 1600) using the sinter program as described in table 1 with a maximum sintering temperature T(max) of 1250°C or 1300°C. The surface of the sintered section was lapped /polished according to DIN EN 843-1:2008-08. The test body had a thickness of 2.0 mm.

1.5 CIE L*a*b* color space and contrast ratio

The CIE color coordinates L*, a* and b* were determined according to DIN 5033 and DIN 6174 and the translucency was determined according to BS 5612. The measurements were carried out using a CM-3700d (Konica-Minolta) type spectrophotometer. Each one of the test bodies was prepared by milling a section from the dental mill blank, followed by sintering the section to full density in a sinter oven, and surface preparation of the sintered section to provide the test body. Surface preparation was carried out by consecutively grinding/polishing both sides of the sintered section with diamond particles of decreasing grain size (35 $\mu$m diamond disc, 20 N, 2 x 6 min; 9 $\mu$m diamond suspension, 10 N, 2-4 x 6 min; 3 $\mu$m diamond suspension, 5 N, 2-4 x 6 min; 1 $\mu$m diamond suspension, 5 N, 2-4 x 6 min). The test body had a final thickness of 1.00 $\pm$ 0.05 mm.

The translucency is calculated from the CR value determined according to BS 5612 according to the formula: translucency [%] = 100% - CR[%].

## 2. Examples

Zirconia powders P1 and P2 were used to prepare dental mill blanks according to embodiments of the present invention. Powder P1 was obtained as a commercial product from Tosoh Corporation, Japan. Properties of powder P1 are shown in table 2. Properties of powder P2 are shown in table 3.

Table 2: properties of zirconia powder P1

| Chemical component | Unit | |
|---|---|---|
| $ZrO_2$ + $HfO_2$ | wt.% | $\geq$ 96.8 |
| $Y_2O_3$ | wt.% | 2.9 |
| $Al_2O_3$ | wt.% | 0.25 |
| Other oxides (e.g., $Fe_2O_3$, $TiO_2$, $SiO_2$, CaO, $Na_2O$) | wt.% | $\leq$ 0.02 |
| **Physical characteristics** | | |
| Bulk density | g/cm$^3$ | 1.4 |
| Specific surface area | m$^2$/g | 16 |
| Crystallite size | nm | 29 |

Table 3: properties of zirconia powder P2

| Chemical component | Unit | |
|---|---|---|
| $ZrO_2$ | wt.% | 94.5 |
| $HfO_2$ | wt% | 1.9 |
| $Y_2O_3$ | wt.% | 3.0 |
| $Al_2O_3$ | wt.% | 0.5 |
| Other oxides (e.g., $Fe_2O_3$, $TiO_2$, $SiO_2$, CaO, $Na_2O$) | wt.% | $\leq 0.02$ |

Zirconia powders P1 and P2 were used to prepare dental mill blanks according to embodiments of the present invention. Powders P1 and P2 were used in binder-free form or in combination with an organic binder. The inventive dental mill blanks were prepared as follows: Powder P1 or powder P2 was axially pre-pressed to a provide a formed body which was subsequently subjected cold isostatic pressing (CIP) to provide a green body. The green body was then subjected to a debinding/pre-sintering step to provide the inventive dental mill blank. When powder P1 and powder P2 were used with a binder, the powder P1 was used with an organic binder in an amount of about 4 wt.% and the powder P2 with an organic binder in an amount of about 3 wt.% (based on the total weight of the powder incl. binder). When powders were used without binder, an amount of about 6 wt% of water was added to the powders to modify the pressing properties. Colorants or other additives were not added. The pressure and debinding/pre-sintering parameters are shown in table 4.

Table 4: pressure and debinding/pre-sintering parameters for preparing dental mill blanks

| Parameters for powders w/o binder | | | | |
|---|---|---|---|---|
| | Pressure | | Pre-sintering | |
| Diameter of mill blank | Axial prepressing | CIP | Maximum temperature T(max) | Holding time at T(max) |
| 40 mm | 20 MPa | 350 MPa | 700 °C | 2 h |
| 100 mm | 40 MPa | 350 MPa | 700 °C | 2 h |
| Parameters for powders with binder | | | | |
| | Pressure | | Debinding/pre-sintering | |
| Diameter of mill blank | Axial prepressing | CIP | Maximum temperature T(max) | Holding time at T(max) |
| 40 mm | 50-80 MPa | 350 MPa | 700 °C | 2 h |
| 100 mm | 60-100 MPa | 350 MPa | 700 °C | 2 h |

The white hardness of the inventive dental mill blanks (hardness of the porous material before densely sintering) was determined for each type of dental mill blanks according to the method described in section "Measuring methods" above and indicated in the table below. The results are shown in table 5 below.

Table 5: white hardness of inventive dental mill blanks

| Powder | Binder | White hardness | |
|---|---|---|---|
| | | [HV2.5] | |
| | | mean | dev. |
| P1 | No | 446 | 14 |
| P1 | Yes | 442 | 23 |
| P2 | No | 802 | 39 |
| P2 | Yes | 481 | 11 |
| | | ISO 14705 | |

The mechanical properties and optical properties of the yttria-containing zirconia material of the inventive dental mill blanks were tested by preparing a densely sintered test body from a section of the mill blank as described in section "Measuring methods" above. The densely sintered test bodies had the properties shown in tables 6.1 to 6.4.

Table 6.1: Vickers hardness of densely sintered test bodies prepared from inventive dental mill blanks

| Powder | Binder | Vickers hardness | | | |
|---|---|---|---|---|---|
| | | [HV20] / [N/mm$^2$] | | | |
| | | Densely sintered at T(max): 1250°C | | Densely sintered at T(max): 1300°C | |
| | | mean | dev. | mean | dev. |
| P1 | No | 12428 | 81 | 12289 | 46 |
| P1 | Yes | 11946 | 194 | 11829 | 70 |
| P2 | No | 11600 | 109 | 11522 | 122 |
| P2 | Yes | 11965 | 80 | 11833 | 104 |
| | | ISO 14705:2008 | | | |

Table 6.2: Fracture resistance of densely sintered test bodies prepared from inventive dental mill blanks

| Po wd er | Bin der | Fracture resistance | | | | Fracture resistance | | | |
|---|---|---|---|---|---|---|---|---|---|
| | | [MN/m$^{-3/2}$] | | | | [MN/m$^{-3/2}$] | | | |
| | | Densely sintered at T(max): 1250°C | | Densely sintered at T(max): 1300°C | | Densely sintered at T(max): 1250°C | | Densely sintered at T(max): 1300°C | |
| | | mean | dev. | mean | dev. | mean | dev. | mean | dev. |
| P1 | No | 13.40 | 0.20 | 14.27 | 0.17 | 16.99 | 0.16 | 17.17 | 0.19 |
| P1 | Yes | 12.52 | 0.37 | 12.44 | 0.28 | 16.07 | 0.31 | 15.99 | 0.23 |
| P2 | No | 15.79 | 0.46 | 13.07 | 0.40 | 18.43 | 0.24 | 16.49 | 0.29 |
| P2 | Yes | 15.63 | 0.66 | 16.30 | 1.10 | 18.29 | 0.37 | 18.57 | 0.50 |
| | | Niihara (radial cracks) | | | | ISO 14627:2012 | | | |

Table 6.3: Biaxial flexural strength of densely sintered test bodies prepared from inventive dental mill blanks

| Powder | Binder | Biaxial flexural strength | | | |
|---|---|---|---|---|---|
| | | [MPa] | | | |
| | | Densely sintered at T(max): 1250°C | | Densely sintered at T(max): 1300°C | |
| | | mean | dev. | mean | dev. |
| P1 | No | 1684 | 211 | 1771 | 186 |
| P1 | Yes | 1490 | 69 | 1505 | 125 |
| P2 | No | 2025 | 111 | 1934 | 111 |
| P2 | Yes | 1940 | 142 | 1814 | 124 |
| | | DIN EN ISO 6872 | | | |

Table 6.4: Optical properties of densely sintered test bodies prepared from inventive dental mill blank

| Powder | Binder | | | Test bodies | |
|---|---|---|---|---|---|
| | | | | densely sintered at T(max) = 1250 °C | densely sintered at T(max) = 1300 °C |
| P1 | No | **Opacity** | Contrast ratio (CR) in [%] | 79.9 | 76.5 |
| | | **Translucency** | [%] | 21.1 | 23.5 |
| | | **Color** | L* | 89.08 | 88.96 |
| | | | a* | -0.59 | -0.61 |
| | | | b* | 1.63 | 1.75 |
| P2 | Yes | **Opacity** | Contrast ratio (CR) in [%] | 83.4 | 84.3 |
| | | **Translucency** | [%] | 16.6 | 15.7 |
| | | **Color** | L* | 89.05 | 89.18 |
| | | | a* | -0.42 | -0.43 |
| | | | b* | 2.09 | 2.02 |

The results shown in tables 5 and 6 show that the inventive dental mill blanks comprises an yttria-containing zirconia material that, when densely sintered, provides high fracture resistance in combination with a high biaxial flexural strength and desirable color properties. Therefore, the inventive dental mill blanks are suitable for preparing a dental restoration which has a high fracture resistance in combination with a high biaxial flexural strength and desirable color properties.

Sintering a test body obtained from the inventive dental mill blanks at maximum sintering temperatures of at most 1350 °C (e.g., at 1250°C and 1300°C) resulted in better material properties, when compared to a maximum sintering temperature of 1400 °C or above.

Known values for mechanical properties of test bodies obtained from prior art dental mill blanks are shown in table 7 for reference.

Table 7: known properties of test bodies obtained from prior art dental mill blanks

| Type of dental mill blank | Properties of test bodies (densely sintered) | | |
|---|---|---|---|
| 3Y-TZP (3 mol%-$Y_2O_3$ stabilized zirconia)* | Biaxial flexural strength | [MPa] | $1000 \pm 200$*** |
| | Fracture resistance | [MPa*m$^{-1/2}$] | $5.25 \pm 0.25$ |
| 5Y-TZP (5 mol%-$Y_2O_3$ stabilized zirconia)** | Biaxial flexural strength | [MPa] | $600 \pm 50$*** |
| | Fracture resistance | [MPa*m$^{-1/2}$] | $2.40 \pm 0.25$ |
| * 4.5-6.0 wt.% $Y_2O_3$, ** 9.0-10.0 wt.% $Y_2O_3$, *** By grinding/polishing specimens, strength values can be increased due to compressive stress formation at surface. | | | |

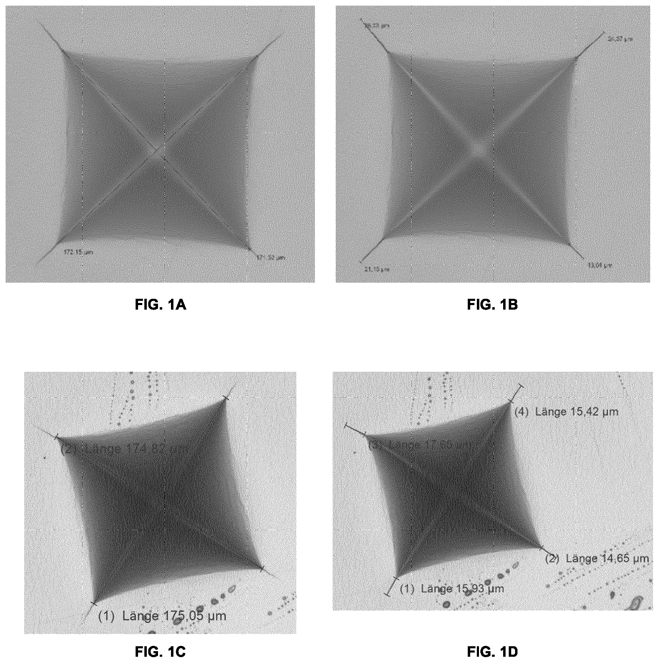

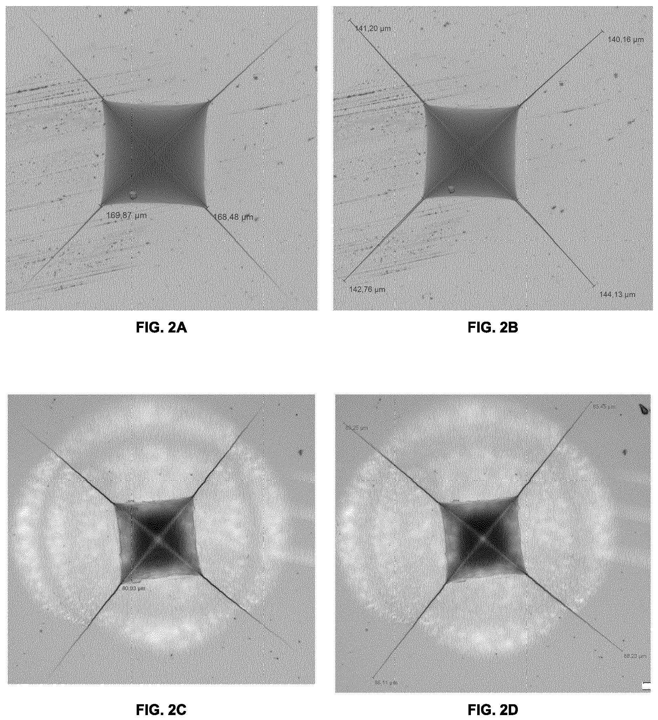

As can be seen from the reference data shown in table 7, the inventive dental mill blanks are suitable for providing a dental restoration with improved mechanical properties. The increased fracture resistance of test bodies prepared from the inventive dental mill blanks are also indicated by the lower crack length after Vickers indentation shown in the exemplary light microscopic images provided in FIGS. 1A-1D, when compared to the crack length for test bodies prepared from the prior art dental mill blanks shown in the exemplary light microscopic images provided in FIGS. 2A-2D.

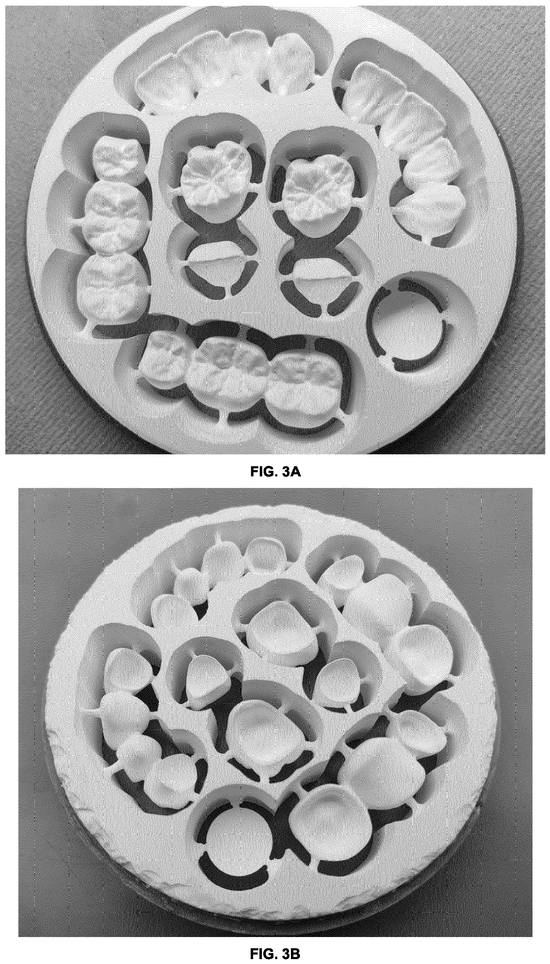

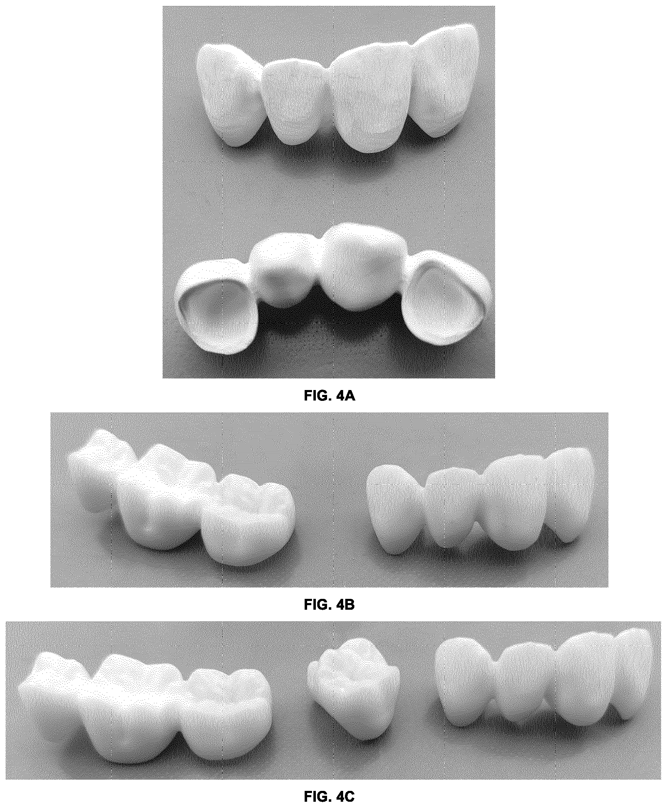

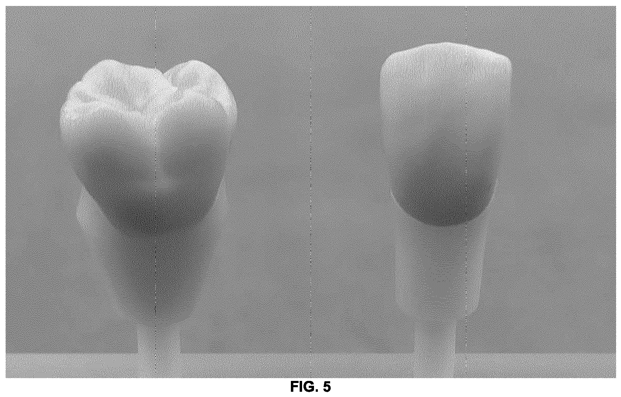

Dental restorations were obtained by milling dental restoration precursors from the inventive dental mill blanks using a CAD/CAM process. The dental restoration precursors were densely sintered at a maximum sintering temperature of 1250°C or 1300°C. Images of the dental restoration precursors and dental restorations are shown in FIGS. 3 to 5. In some instances, the dental restoration precursor was colored using a brush infiltration technique with a commercial coloring liquid (see FIG. 5).

**Claims**

1. A dental mill blank comprising an yttria-containing zirconia material, the yttria-containing zirconia material having:

   - a fracture resistance of at least 6.0 MPa*m$^{-1/2}$, determined on a densely sintered test body of the yttria-containing zirconia material, and
   - a biaxial flexural strength of at least 1200 MPa, determined on a densely sintered test body of the yttria-containing zirconia material.

2. The dental mill blank according to claim 1, the yttria-containing zirconia material comprising yttria in an amount of at most 4.0 wt.%, preferably in an range of 1.5 to 4.0 wt.%, and more preferably in a range of 2.0 to 3.8 wt.%, like in a range of 2.2 to 3.4 wt.%, based on the total weight of the yttria-containing zirconia material.

3. The dental mill blank according to any one of the preceding claims, the yttria-containing zirconia material comprising aluminum oxide in an amount of at most 2.0 wt.%, preferably in a range of 0.0 to 1.5 wt.%, more preferably in a range of 0.0 to 1.0 wt.%, and even more preferably in a range of 0.0 to 0.7 wt.%, like in a range of 0.0 to 0.5 wt.%, 0.1 to 0.4 wt.% or 0.4 to 0.7 wt.%, based on the total weight of the yttria-containing zirconia material.

4. The dental mill blank according to any one of the preceding claims, the yttria-containing zirconia material comprising

   from 92.5 to 98.5 wt.%, preferably from 93.2 to 98.0 wt.%, like from 94.0 to 98.0 wt.%, of a combined amount of zirconia and hafnia,
   from 1.5 to 4.0 wt.%, preferably from 2.0 to 3.8 wt.%, like from 2.2 to 3.4 wt.%, of yttria,
   from 0.0 to 1.5 wt.%, preferably from 0.0 to 1.0 wt.%, like from 0.1 to 0.7 wt.% of aluminum oxide, and
   from 0.0 to 5.0 wt.%, preferably from 0.0 to 4.5 wt.%, like from 0.0 to 3.0 wt.%, of other oxides which optionally comprise coloring metal oxides,
   based on the total weight of the yttria-containing zirconia material.

5. The dental mill blank according to claim 4, the yttria-containing zirconia material comprising

   from 93.0 to 98.5 wt.%, preferably from 94.0 to 98.0 wt.%, like from 95.0 to 97.7 wt.%, of a combined amount of zirconia and hafnia,
   from 1.5 to 4.0 wt.%, preferably from 2.0 to 3.8 wt.%, like from 2.2 to 3.4 wt.%, of yttria,
   from 0.0 to 1.0 wt.%, preferably from 0.0 to 0.7 wt.%, like from 0.1 to 0.7 wt.% of aluminum oxide (e.g., from 0.1 to 0.4 wt.% or from 0.4 to 0.7 wt.%), and
   from 0.0 to 2.0 wt.%, preferably from 0.0 to 1.5 wt.%, like from 0.0 to 1.0 wt.%, of one or more coloring metal oxides, optionally comprising iron oxide, erbium oxide, chromium oxide, manganese oxide, terbium oxide, praseodymium oxide, or any combination thereof, and
   optionally less than 0.2 wt.% of other oxides (e.g., $TiO_2$, $SiO_2$, CaO, and/or $Na_2O$).

6. The dental mill blank according to any one of the preceding claims, the yttria-containing zirconia material having a fracture resistance of at least 8.0 MPa*m$^{-1/2}$, and preferably at least 10.0 MPa*m$^{-1/2}$, like at least 12.0 MPa*m$^{-1/2}$, determined on a densely sintered test body of the yttria-containing zirconia material.

7. The dental mill blank according to any one of the preceding claims, the yttria-containing zirconia material having a biaxial flexural strength of at least 1300 MPa, and preferably at least 1350 MPa, like at least 1400 MPa, determined on a densely sintered test body of the yttria-containing zirconia material.

8. The dental mill blank according to any one of the preceding claims, the yttria-containing zirconia material having:

   - a contrast ratio of less than 90%, and/or
   - CIE L*a*b* color values of:

     L* in range of 76 to 96,
     a* in a range of (-2) to 6,
     b* in a range of 0 to 25,

   wherein the contrast ratio and/or CIE L*a*b* color values are determined on a densely sintered test body of the yttria-containing zirconia material having a thickness of 1.00 mm $\pm$ 0.05.

9.  The dental mill blank according to any one of the preceding claims, the yttria-containing zirconia material having a Vickers hardness VH of at least 9 000 MPa, and preferably at least 10 000 MPa, like at least 11 000 MPa, determined on a densely sintered test body of the yttria-containing zirconia material.

10. The dental mill blank according to any one of the preceding claims, having a white hardness, determined according to ISO14705 using a test load of 24.5 N, of at least 250 MPa, and preferably at least 300 MPa, like at least 350 MPa or at least 400 MPa.

11. The dental mill blank according to any one of the preceding claims, being composed of the yttria-containing zirconia material, or
    comprising the yttria-containing zirconia material in form of a subsection of the dental mill blank.

12. A process for preparing a dental mill blank according to any one of claims 1 to 11 comprising the steps of:

    - providing a zirconia powder,
    - optionally treating the zirconia powder with one or more colorants,
    - compacting the zirconia powder to provide a green body;
    - pre-sintering the green body to provide a dental mill blank, and
    - optionally pre-coloring the dental mill blank.

13. The process according to claim 12, wherein the provided zirconia powder comprises from 95.0 to 98.5 wt.%, preferably from 95.5 to 98.0 wt.%, like from 96.0 to 97.7 wt.%, of a combined amount of zirconia and hafnia,

    from 1.5 to 4.0 wt.%, preferably from 2.0 to 3.8 wt.%, like from 2.2 to 3.4 wt.%, of yttria,
    from 0.0 to 1.0 wt.%, preferably from 0.0 to 0.7 wt.%, like from 0.1 to 0.7 wt.% of aluminum oxide, and
    from 0.0 to 0.3 wt.%, preferably from 0.0 to 0.1 wt.%, like from 0.0 to 0.05 wt.%, of other oxides, based on the total weight of the zirconia powder.

14. A process for preparing a dental restoration, the process comprising the steps of:

    - machining a dental mill blank according to any one of claims 1 to 11 to provide a dental restoration precursor;
    - optionally surface-treating the dental restoration precursor;
    - sintering the dental restoration precursor to provide a dental restoration.

15. The process according to claim 14, wherein the sintering of the dental restoration precursor is carried out at a maximum sintering temperature of at most 1350°C, like in a range of 1200 to 1350°C.

FIG. 1A

FIG. 1B

FIG. 1C

FIG. 1D

FIG. 2A

FIG. 2B

FIG. 2C

FIG. 2D

FIG. 3A

FIG. 3B

FIG. 4A

FIG. 4B

FIG. 4C

FIG. 5

Europäisches
Patentamt

European
Patent Office

Office européen
des brevets

# EUROPEAN SEARCH REPORT

**Application Number**

EP 23 21 8110

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/081369 A1 (KIM JAE WON [US] ET AL) 17 March 2022 (2022-03-17) * Sample 15E; paragraphs [0132] - [0134]; claims 1-18; figure 15; examples 22-24 * | 1-15 | INV. A61C13/083 C04B35/488 C04B35/626 C04B38/00 C04B35/486 |
| X | US 11 180 417 B2 (3M INNOVATIVE PROPERTIES CO [US]) 23 November 2021 (2021-11-23) * claims 1-9; examples CE1, CE2, EX2; tables 5, 7 * | 1-15 | |
| A | US 2020/405586 A1 (HAUPTMANN HOLGER [DE] ET AL) 31 December 2020 (2020-12-31) * paragraphs [0258] - [0281]; claims 1-14 * | 1-15 | |
| A | US 2023/338123 A1 (JAHNS MICHAEL [DE] ET AL) 26 October 2023 (2023-10-26) * claims 1-15 * | 1-15 | |

**TECHNICAL FIELDS SEARCHED (IPC)**

A61C
C04B

The present search report has been drawn up for all claims

| Place of search | Date of completion of the search | Examiner |
|---|---|---|
| The Hague | 6 June 2024 | Süzük, Kerem Güney |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 23 21 8110

This annex lists the patent family members relating to the patent documents cited in the above-mentioned European search report.
The members are as contained in the European Patent Office EDP file on
The European Patent Office is in no way liable for these particulars which are merely given for the purpose of information.

06-06-2024

| Patent document cited in search report | Publication date | Patent family member(s) | | Publication date |
|---|---|---|---|---|
| US 2022081369 A1 | 17-03-2022 | NONE | | |
| US 11180417 B2 | 23-11-2021 | EP | 3583083 A1 | 25-12-2019 |
| | | US | 2020055779 A1 | 20-02-2020 |
| | | WO | 2018151995 A1 | 23-08-2018 |
| US 2020405586 A1 | 31-12-2020 | CN | 111801067 A | 20-10-2020 |
| | | EP | 3758645 A1 | 06-01-2021 |
| | | JP | 7407722 B2 | 04-01-2024 |
| | | JP | 2021515754 A | 24-06-2021 |
| | | JP | 2024009816 A | 23-01-2024 |
| | | US | 2020405586 A1 | 31-12-2020 |
| | | WO | 2019166920 A1 | 06-09-2019 |
| US 2023338123 A1 | 26-10-2023 | CN | 116457200 A | 18-07-2023 |
| | | EP | 4225579 A1 | 16-08-2023 |
| | | JP | 2023546824 A | 08-11-2023 |
| | | US | 2023338123 A1 | 26-10-2023 |
| | | WO | 2022074494 A1 | 14-04-2022 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2707342 B1

**Non-patent literature cited in the description**

- Mechanical Properties, Failure Behaviour, Materials Selection. **D. MUNZ** ; **T. FETT**. Ceramics. Springer Verlag, 1999, 34-37